# EUROPEAN PATENT APPLICATION

(11) **EP 3 842 491 A1**
(43) Date of publication of application: **30.06.2021**
(21) Application number: 19851818.5
(22) Date of filing: 21.08.2019
(51) Int. Cl.: C08L 61/04, C07C 39/367, C07C 69/712, C07C 69/96, C08G 8/00, G03F 7/004, G03F 7/038, G03F 7/039

(54) **COMPOUND, COMPOSITION CONTAINING THE SAME, METHOD FOR FORMING RESIST PATTERN, AND METHOD FOR FORMING INSULATING FILM**

(30) Priority: 24.08.2018 JP 2018157622
(71) Applicant: MITSUBISHI GAS CHEMICAL COMPANY, INC., Chiyoda-ku Tokyo 100-8324 (JP)
(72) Inventor: SATO, Takashi, Hiratsuka-shi, Kanagawa 254-0016 (JP); ECHIGO, Masatoshi, Tokyo 100-8324 (JP); MAKINOSHIMA, Takashi, Hiratsuka-shi, Kanagawa 254-0016 (JP)
(74) Representative: Müller-Boré & Partner Patentanwälte PartG mbB
(86) International application number: PCT/JP2019/032528
(87) International publication number: WO 2020/040162

(57) **Abstract**

A composition comprising a polyphenol compound (B),
wherein the polyphenol compound (B) is one or more selected from the group consisting of a compound represented by the following formula (1) and a resin having a structure represented by the following formula (2) :

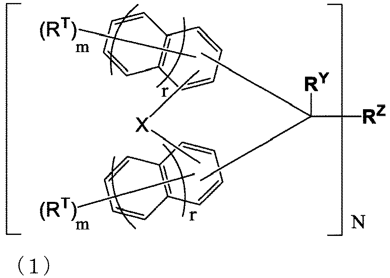

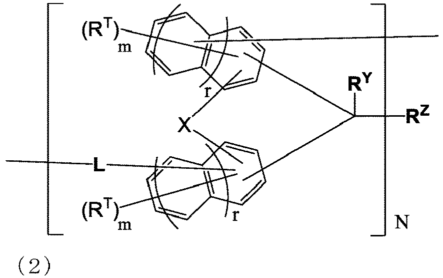

## Description

### Technical Field

The present invention relates to a novel compound, a composition containing the same, as well as a method for forming a resist pattern and a method for forming an insulating film, and in particular, relates to a composition used for film formation purposes for lithography and film formation purposes for resist, and a film formation method using the same.

### Background Art

In recent years, in the production of semiconductor elements and liquid crystal display elements, semiconductors (patterns) and pixels have been rapidly miniaturized due to the advance in lithography technology. As an approach for pixel miniaturization, the exposure light source has been shifted to have a shorter wavelength, in general. Specifically, ultraviolet rays typified by g-ray and i-ray have been used conventionally, but nowadays, far ultraviolet exposure such as KrF excimer laser (248 nm) and ArF excimer laser (193 nm) is being the center of mass production. Furthermore, the introduction of extreme ultraviolet (EUV) lithography (13.5 nm) is progressing. In addition, electron beam (EB) is also used for forming a fine pattern.

Up to now, typical resist materials are polymer based resist materials capable of forming an amorphous film. Examples include polymer based resist materials such as polymethyl methacrylate, polyhydroxy styrene with an acid dissociation group, and polyalkyl methacrylate (see, for example, Non Patent Literature 1).

Conventionally, a line pattern of about 10 to 100 nm is formed by irradiating a resist thin film made by coating a substrate with a solution of these resist materials with ultraviolet, far ultraviolet, electron beam, extreme ultraviolet or the like.

In addition, lithography using electron beam or extreme ultraviolet has a reaction mechanism different from that of normal photolithography. Furthermore, lithography with electron beam or extreme ultraviolet aims at forming fine patterns of several nm to ten-odd nm. Accordingly, there is a demand for a resist material having higher sensitivity for an exposing source when the resist pattern dimension is reduced. In particular, lithography with extreme ultraviolet is required to further increase sensitivity in terms of throughput.

As a resist material that solves the problems as mentioned above, an inorganic resist material having a metallic element such as titanium, tin, hafnium and zirconium has been proposed (see, for example, Patent Literature 1).

### Citation List

### Patent Literature

Patent Literature 1: Japanese Patent Laid-Open No. 2015-108781

### Non Patent Literature

Non Patent Literature 1: Shinji Okazaki et al., "Development of Lithography Technology in These 40 Years", S&T Publishing Inc., December 9, 2016

### Summary of Invention

### Technical Problem

However, conventionally developed resist compositions have problems such as many film defects, insufficient sensitivity, insufficient etching resistance, or poor resist pattern. In particular, for producing a 3D NAND device, it is necessary to form a thick resist, and there is an urgent need to solve these problems (in particular, insufficient etching resistance).

In light of the circumstances described above, an object of the present invention is to provide a composition capable of forming a film having high etching resistance, as well as a method for forming a resist pattern and a method for forming an insulating film, using the composition.

### Solution to Problem

The inventors have, as a result of devoted examinations to solve the problems mentioned above, found out that a compound and resin having a specific structure have high solubility in a safe solvent, and are capable of forming a film having high etching resistance when the compound and the like are used in a composition for film formation purposes for photography or film formation purposes for resist, leading to completion of the present invention.

More specifically, the present invention is as follows.
[1] A composition comprising a polyphenol compound (B),
   wherein the polyphenol compound (B) is one or more selected from the group consisting of a compound represented by the following formula (1) and a resin having a structure represented by the following formula (2) : 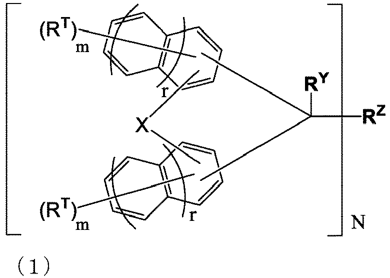
   wherein R^{Y} is a hydrogen atom, an alkyl group having 1 to 30 carbon atoms and optionally having a substituent, or an aryl group having 6 to 30 carbon atoms and optionally having a substituent;
   R^{Z} is an N-valent group having 1 to 60 carbon atoms and optionally having a substituent, or a single bond;
   each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
   R^{z} contains at least two iodine atoms;
   X is an oxygen atom, a sulfur atom or not a crosslink;
   each m is independently an integer of 0 to 9 (provided that at least one m is an integer of 1 to 9);
   each r is independently an integer of 0 to 2 (provided that at least one r is an integer of 1 to 2); and
   N is an integer of 1 to 4 (provided that when N is an integer of 2 or larger, N structural formulas within the parentheses [ ] are the same or different), and

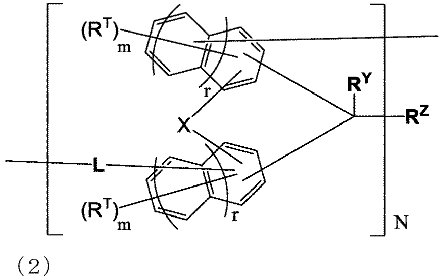

   wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
   R^{Y} is a hydrogen atom, an alkyl group having 1 to 30 carbon atoms and optionally having a substituent, or an aryl group having 6 to 30 carbon atoms and optionally having a substituent;
   R^{Z} is an N-valent group having 1 to 60 carbon atoms and optionally having a substituent, or a single bond;
   each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
   R^{z} contains at least two iodine atoms;
   X is an oxygen atom, a sulfur atom or not a crosslink;
   each m is independently an integer of 0 to 9 (provided that at least one m is an integer of 1 to 9);
   each r is independently an integer of 0 to 2 (provided that at least one r is an integer of 1 to 2); and
   N is an integer of 1 to 4 (provided that when N is an integer of 2 or larger, N structural formulas within the parentheses [ ] are the same or different).
[2] The composition according to the above [1], further comprising a base material (A), wherein the base material (A) is one or more selected from the group consisting of a phenol novolac resin, a cresol novolac resin, a hydroxystyrene resin, a (meth)acrylic resin, a hydroxystyrene-(meth)acrylic copolymer, a cycloolefin-maleic anhydride copolymer, a cycloolefin, a vinyl ether-maleic anhydride copolymer and an inorganic resist material having a metallic element, and a derivative thereof.
[3]
   The composition according to the above [1] or [2], wherein the polyphenol compound (B) is one or more selected from the group consisting of a compound represented by the following formula (1A) and a resin having a structure represented by the following formula (2A) : 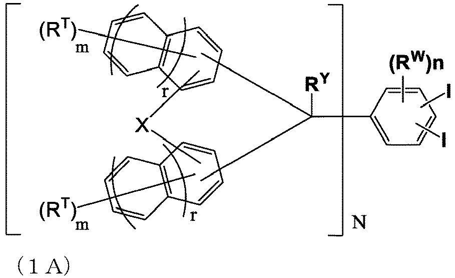
   wherein R^{Y} is a hydrogen atom, an alkyl group having 1 to 30 carbon atoms and optionally having a substituent, or an aryl group having 6 to 30 carbon atoms and optionally having a substituent;
   R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
   X is an oxygen atom, a sulfur atom or not a crosslink;
   each m is independently an integer of 0 to 9 (provided that at least one m is an integer of 1 to 9);
   each r is independently an integer of 0 to 2 (provided that at least one r is an integer of 1 to 2);
   each n is independently an integer of 0 to 3; and
   N is an integer of 1 to 4 (provided that when N is an integer of 2 or larger, N structural formulas within the parentheses [ ] are the same or different), and

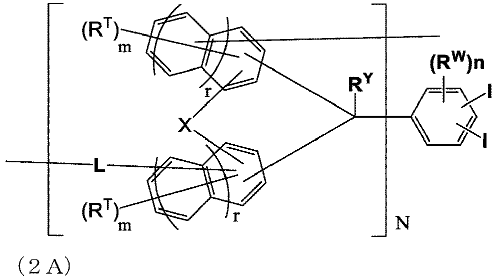

   wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
   R^{Y} is a hydrogen atom, an alkyl group having 1 to 30 carbon atoms and optionally having a substituent, or an aryl group having 6 to 30 carbon atoms and optionally having a substituent;
   R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
   X is an oxygen atom, a sulfur atom or not a crosslink;
   each m is independently an integer of 0 to 9 (provided that at least one m is an integer of 1 to 9);
   each r is independently an integer of 0 to 2 (provided that at least one r is an integer of 1 to 2);
   each n is independently an integer of 0 to 3; and
   N is an integer of 1 to 4 (provided that when N is an integer of 2 or larger, N structural formulas within the parentheses [ ] are the same or different).
[4] The composition according to the above [2] or [3], wherein a mass ratio between the base material (A) and the polyphenol compound (B) is 5:95 to 95:5.
[5] The composition according to any one of the above [1] to [4], further comprising a solvent.
[6] The composition according to any one of the above [1] to [5], further comprising an acid generating agent.
[7] The composition according to any one of the above [1] to [6], further comprising a crosslinking agent.
[8] The composition according to any one of the above [1] to [7], wherein the composition is used in film formation for lithography.
[9] The composition according to any one of the above [1] to [7], wherein the composition is used in film formation for resist.
[10] A method for forming a resist pattern, comprising the steps of:
   forming a photoresist layer on a substrate using the composition according to any one of the above [1] to [7];
   irradiating a predetermined region of the photoresist layer formed on the substrate with radiation; and
   developing the photoresist layer after the radiation irradiation.
[11] A method for forming an insulating film, comprising the steps of:
   forming a photoresist layer on a substrate using the composition according to any one of the above [1] to [7];
   irradiating a predetermined region of the photoresist layer formed on the substrate with radiation; and
   developing the photoresist layer after the radiation irradiation.
[12] A compound represented by the following formula (1): 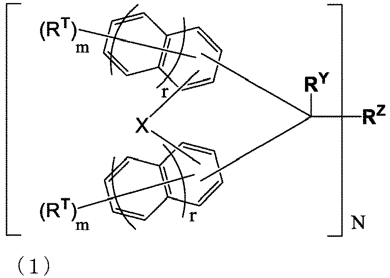
   wherein R^{Y} is a hydrogen atom, an alkyl group having 1 to 30 carbon atoms and optionally having a substituent, or an aryl group having 6 to 30 carbon atoms and optionally having a substituent;
   R^{Z} is an N-valent group having 1 to 60 carbon atoms and optionally having a substituent, or a single bond;
   each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
   R^{z} contains at least two iodine atoms;
   X is an oxygen atom, a sulfur atom or not a crosslink;
   each r is independently an integer of 0 to 2 (provided that at least one r is an integer of 1 to 2);
   each m is independently an integer of 0 to 9 (provided that at least one m is an integer of 1 to 9); and
   N is an integer of 1 to 4 (provided that when N is an integer of 2 or larger, N structural formulas within the parentheses [ ] are the same or different).
[13] A resin having a structure represented by the following formula (2): 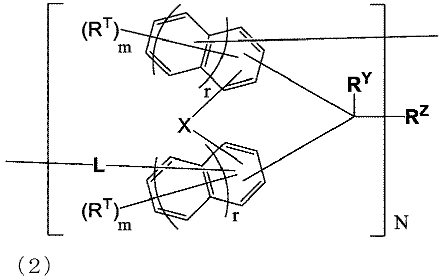
   wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
   R^{Y} is a hydrogen atom, an alkyl group having 1 to 30 carbon atoms and optionally having a substituent, or an aryl group having 6 to 30 carbon atoms and optionally having a substituent;
   R^{Z} is an N-valent group having 1 to 60 carbon atoms and optionally having a substituent, or a single bond;
   each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
   R^{z} contains at least two iodine atoms;
   X is an oxygen atom, a sulfur atom or not a crosslink;
   each m is independently an integer of 0 to 9 (provided that at least one m is an integer of 1 to 9);
   each r is independently an integer of 0 to 2 (provided that at least one r is an integer of 1 to 2); and
   N is an integer of 1 to 4 (provided that when N is an integer of 2 or larger, N structural formulas within the parentheses [ ] are the same or different).
[14] A compound represented by the following formula (1A) : 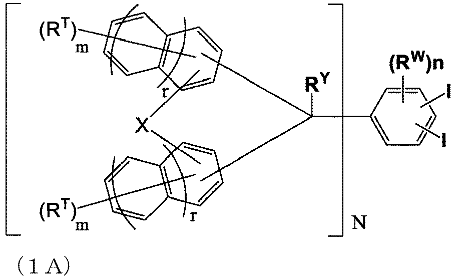
   wherein R^{Y} is a hydrogen atom, an alkyl group having 1 to 30 carbon atoms and optionally having a substituent, or an aryl group having 6 to 30 carbon atoms and optionally having a substituent;
   R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
   X is an oxygen atom, a sulfur atom or not a crosslink;
   each m is independently an integer of 0 to 9 (provided that at least one m is an integer of 1 to 9);
   each r is independently an integer of 0 to 2 (provided that at least one r is an integer of 1 to 2);
   each n is independently an integer of 0 to 3; and
   N is an integer of 1 to 4 (provided that when N is an integer of 2 or larger, N structural formulas within the parentheses [ ] are the same or different).
[15] A resin having a structure represented by the following formula (2A): 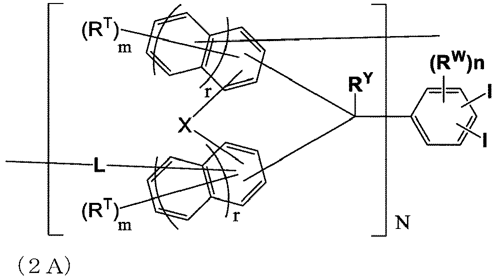
   wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
   R^{Y} is a hydrogen atom, an alkyl group having 1 to 30 carbon atoms and optionally having a substituent, or an aryl group having 6 to 30 carbon atoms and optionally having a substituent;
   R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
   X is an oxygen atom, a sulfur atom or not a crosslink;
   each m is independently an integer of 0 to 9 (provided that at least one m is an integer of 1 to 9);
   each r is independently an integer of 0 to 2 (provided that at least one r is an integer of 1 to 2); each n is independently an integer of 0 to 3; and
   N is an integer of 1 to 4 (provided that when N is an integer of 2 or larger, N structural formulas within the parentheses [ ] are the same or different).
[16] A compound represented by the following formula (1B) : 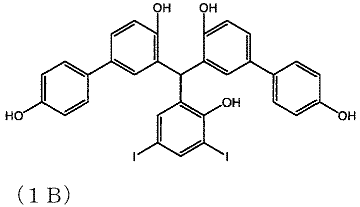
[17] A compound represented by the following formula (2B) :

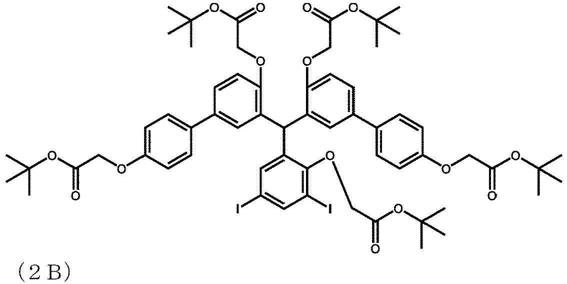

### Advantageous Effects of Invention

According to the present invention, it is possible to provide a composition capable of forming a film having high etching resistance, as well as a method for forming a resist pattern and a method for forming an insulating film, using the composition.

### Description of Embodiments

Hereinafter, an embodiment of the present invention will be described (hereinafter, may be referred to as the "present embodiment"). The present embodiment is given in order to illustrate the present invention. The present invention is not limited to only the present embodiment.

The composition of the present embodiment is a composition that contains a polyphenol compound (B) containing two or more iodine atoms and is particularly suitable for lithography technology, and can be used for, without particular limitation, film formation purposes for lithography, for example, resist film formation purposes (that is, a "composition for resist film formation"). Furthermore, the composition of the present embodiment can be used for upper layer film formation purposes (that is, a "composition for upper layer film formation"), intermediate layer formation purposes (that is, a "composition for intermediate layer formation"), underlayer film formation purposes (that is, a "composition for underlayer film formation"), and the like. According to the composition of the present embodiment, a film having high sensitivity can be formed and a good shape can be imparted to a resist pattern.

The composition of the present embodiment can also be used as an optical component forming composition applying lithography technology. The optical component is used in the form of a film or a sheet and is also useful as a plastic lens (a prism lens, a lenticular lens, a microlens, a Fresnel lens, a viewing angle control lens, a contrast improving lens, etc.), a phase difference film, a film for electromagnetic wave shielding, a prism, an optical fiber, a solder resist for flexible printed wiring, a plating resist, an interlayer insulating film for multilayer printed circuit boards, a photosensitive optical waveguide, a liquid crystal display, an organic electroluminescent (EL) display, an optical semiconductor (LED) element, a solid state image sensing element, an organic thin film solar cell, a dye sensitized solar cell, and an organic thin film transistor (TFT). It can be particularly suitably utilized as an embedded film and a smoothed film on a photodiode, a smoothed film in front of or behind a color filter, a microlens, and a smoothed film and a conformal film on a microlens, all of which are components of a solid state image sensing element, to which high refractive index is demanded.

### <<Composition>>

The composition of the present embodiment contains the polyphenol compound (B) containing two or more iodine atoms, and may also contain other components such as a base material (A), a solvent (S), an acid generating agent (C), a crosslinking agent (G), and an acid diffusion controlling agent (E), in addition to the polyphenol compound (B), if required. Hereinafter, each of these components will be described.

### [Base material (A)]

The "base material (A)" in the present embodiment is a compound (including a resin) other than the polyphenol compound, which will be mentioned later, and means a base material applied as a resist for, for example, g-ray, i-ray, KrF excimer laser (248 nm), ArF excimer laser (193 nm), extreme ultraviolet (EUV) lithography (13.5 nm) or electron beam (EB) (for example, a base material for lithography or a base material for resist). These base materials can be used as the base material (A) according to the present embodiment without particular limitation. Examples of the base material (A) include a phenol novolac resin, a cresol novolac resin, a hydroxystyrene resin, a (meth)acrylic resin, and a hydroxystyrene-(meth)acrylic copolymer, a cycloolefin-maleic anhydride copolymer, a cycloolefin, a vinyl ether-maleic anhydride copolymer, and an inorganic resist material having a metallic element such as titanium, tin, hafnium and zirconium, and a derivative thereof. Among them, from the viewpoint of the shape of a resist pattern to be obtained, preferable are a phenol novolac resin, a cresol novolac resin, a hydroxystyrene resin, a (meth)acrylic resin, a hydroxystyrene-(meth)acrylic resin copolymer, and an inorganic resist material having a metallic element such as titanium, tin, hafnium and zirconium, and a derivative thereof.

Examples of the derivative include, but not particularly limited to, those to which a dissociation group is introduced and those to which a crosslinkable group is introduced. The derivative to which a dissociation group or a crosslinkable group is introduced can exhibit dissociation reaction or crosslinking reaction through the effect of light, acid or the like. Examples of the dissociation group and the crosslinkable group can include the same as those described in the polyphenol compound (B), which will be mentioned later.

In the present embodiment, the weight average molecular weight of the base material (A) is preferably 200 to 4990, more preferably 200 to 2990, and still more preferably 200 to 1490 from the viewpoints of reducing defects in a film to be formed by using the composition and of a good pattern shape. As the weight average molecular weight, a value obtained by measuring the weight average molecular weight in terms of polystyrene, using GPC, can be used.

In the present embodiment, the mass ratio between the base material (A) and the polyphenol compound (B) [base material (A):polyphenol compound (B)] is preferably 5:95 to 95:5. When the mass ratio between the base material (A) and the polyphenol compound (B) is within the range, sensitivity is improved and defects in the film are decreased, so that a resist pattern to be obtained tends to be good. Also, the mass ratio is preferably 31:69 to 95:5, and still more preferably 51:49 to 95:5 from the viewpoint of reducing defects.

### [Polyphenol compound (B)]

In the present embodiment, the polyphenol compound (B) is a compound having a plurality of iodine atoms and having any of a phenolic hydroxy group, a crosslinkable group or a dissociation group in the molecule. The polyphenol compound (B) is one or more selected from the group consisting of a compound represented by the following formula (1) and a resin having a structure represented by the following formula (2): 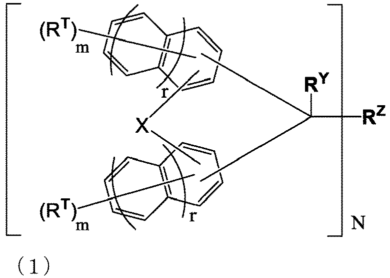
wherein R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms and optionally having a substituent, or a single bond;
each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
R^{z} contains at least two iodine atoms;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2); and
N is an integer of 1 to 4 (provided that when N is an integer of 2 or larger, N structural formulas within the parentheses [ ] are the same or different), and

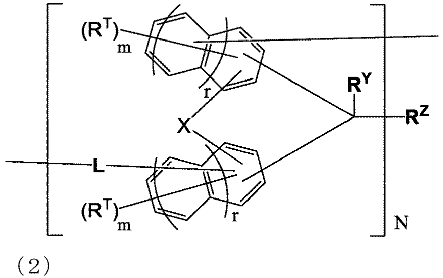

wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms and optionally having a substituent, or a single bond;
each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
R^{z} contains at least two iodine atoms;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2); and
N is an integer of 1 to 4 (provided that when N is an integer of 2 or larger, N structural formulas within the parentheses [ ] are the same or different).

The polyphenol compound (B) has high solubility in a solvent, high heat resistance, low glass transition temperature, low molecular weight, and high etching resistance, and is therefore suitably used in a resist composition having high storage stability or the like that is capable of reducing film defects, has high sensitivity, and can impart a good shape to a resist pattern. In addition, since the affinity with the base material (A) thereof is also high, it can also be used as a sensitizing agent of a resist.

From the viewpoint of easy availability of the objective compound, the polyphenol compound (B) is preferably one or more selected from the group consisting of a compound represented by the following formula (1A) and a resin having a structure represented by the following formula (2A): 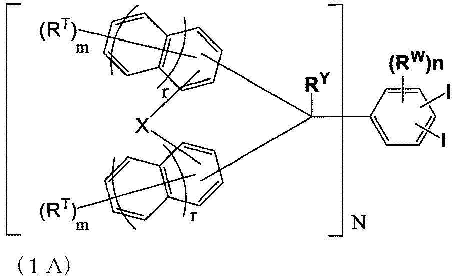
wherein R^{Y} is a hydrogen atom, an alkyl group having 1 to 30 carbon atoms and optionally having a substituent, or an aryl group having 6 to 30 carbon atoms and optionally having a substituent;
R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2);
each n is independently an integer of 0 to 3; and
N is an integer of 1 to 4 (provided that when N is an integer of 2 or larger, N structural formulas within the parentheses [ ] are the same or different), and

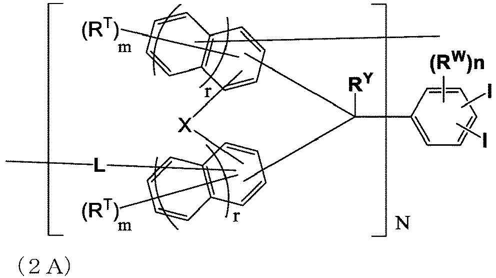

wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 30 carbon atoms and optionally having a substituent;
R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2); each n is independently an integer of 0 to 3; and
N is an integer of 1 to 4 (provided that when N is an integer of 2 or larger, N structural formulas within the parentheses [ ] are the same or different).

The polyphenol compound (B) according to the present embodiment has two or more iodine atoms. When the polyphenol compound (B) has two or more groups having an iodine atom, there is a tendency that absorption of extreme ultraviolet (EUV) can be increased and a sensitization effect of EUV can be exhibited. In addition, from the viewpoint of further increasing the sensitivity, the polyphenol (B) may have a group containing a fluorine atom and/or a group containing a bromine atom in addition to the group containing two or more iodine atoms.

Examples of the group containing an iodine atom include, but not particularly limited to, a linear hydrocarbon group having 1 to 30 carbon atoms and substituted with an iodine atom, a branched hydrocarbon group having 3 to 30 carbon atoms and substituted with an iodine atom, an alicyclic hydrocarbon group having 3 to 30 carbon atoms and substituted with an iodine atom, an aromatic group having 6 to 30 carbon atoms and substituted with an iodine atom, and a group having an aromatic group having 6 to 30 carbon atoms and substituted with an iodine atom. Among the above, from the viewpoint of heat resistance, a branched hydrocarbon group having 3 to 30 carbon atoms and substituted with an iodine atom, an alicyclic hydrocarbon group having 3 to 30 carbon atoms and substituted with an iodine atom, an aromatic group having 6 to 30 carbon atoms and substituted with an iodine atom, or a group having an aromatic group having 6 to 30 carbon atoms and substituted with an iodine atom is preferable; an alicyclic hydrocarbon group having 3 to 30 carbon atoms and substituted with an iodine atom, an aromatic group having 6 to 30 carbon atoms and substituted with an iodine atom, or a group having an aromatic group having 6 to 30 carbon atoms and substituted with an iodine atom is more preferable; and a group having an aromatic group having 6 to 30 carbon atoms and substituted with an iodine atom is still more preferable.

Preferred examples of the group containing the iodine atom include an iodine group, an iodomethyl group, a diiodomethyl group, a diiodophenyl group, a triiodomethyl group, a diiodomethylene group, a hydroxyhexaiodopropyl group, and a hydroxydiiodophenyl group.

In the present specification, unless otherwise defined, the term "substituted" means that one or more hydrogen atoms in a functional group are substituted with a substituent. Examples of the "substituent" include, but not particularly limited to, a halogen atom, a hydroxy group, a cyano group, a nitro group, an amino group, a thiol group, a heterocyclic group, a linear aliphatic hydrocarbon group having 1 to 20 carbon atoms, a branched aliphatic hydrocarbon group having 3 to 20 carbon atoms, a cyclic aliphatic hydrocarbon group having 3 to 20 carbon atoms, an aryl group having 6 to 20 carbon atoms, an aralkyl group having 7 to 30 carbon atoms, an alkoxy group having 1 to 20 carbon atoms, an amino group having 0 to 20 carbon atoms, an alkenyl group having 2 to 20 carbon atoms, an alkynyl group having 2 to 20 carbon atoms, an acyl group having 1 to 20 carbon atoms, an alkoxycarbonyl group having 2 to 20 carbon atoms, an alkyloyloxy group having 1 to 20 carbon atoms, an aryloyloxy group having 7 to 30 carbon atoms, and an alkylsilyl group having 1 to 20 carbon atoms.

It is preferable that the polyphenol compound (B) according to the present embodiment be derivatized for use. There is a tendency that an even better shape can be imparted to a resist pattern through such derivatization. The derivative in the derivatization is not particularly limited, but examples thereof include one in which a crosslinkable group is introduced or one in which a dissociation group is introduced, and the polyphenol compound (B) into which these groups are introduced can exhibit a crosslinking reaction or a dissociation reaction by the action of light, acid, or the like.

The "crosslinkable group" refers to a group that crosslinks in the presence of a catalyst or without a catalyst. Examples of the crosslinkable group include, but not particularly limited to, an alkoxy group having 1 to 20 carbon atoms, a group having an allyl group, a (meth)acryloyl group, a group having an epoxy (meth)acryloyl group, a group having a urethane (meth)acryloyl group, a group having a hydroxy group, a group having a glycidyl group, a group having a vinyl containing phenylmethyl group, a group having a styrene group, a group having an alkynyl group, a group having a carbon-carbon double bond, a group having a carbon-carbon triple bond, and a group containing these groups.

Examples of the group having an allyl group include, but not particularly limited to, a group represented by the following formula (X-1): 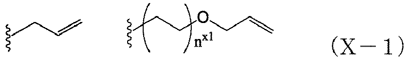 wherein n^{x1} is an integer of 1 to 5.

Examples of the group having a (meth)acryloyl group include, but not particularly limited to, a group represented by the following formula (X-2): 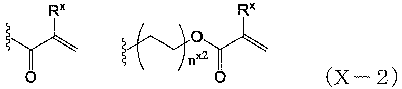 wherein n^{x2} is an integer of 1 to 5, and R^{X} is a hydrogen atom or a methyl group.

Examples of the group having an epoxy (meth)acryloyl group include, but not particularly limited to, a group represented by the following formula (X-3), where the epoxy (meth)acryloyl group refers to a group generated through a reaction between an epoxy (meth)acrylate and a hydroxy group: 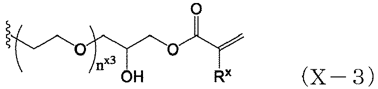 wherein n^{x3} is an integer of 0 to 5, and R^{X} is a hydrogen atom or a methyl group.

Examples of the group having a urethane (meth)acryloyl group include, but not particularly limited to, a group represented by the following formula (X-4) : 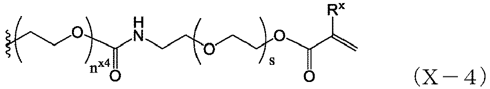 wherein n^{x4} is an integer of 0 to 5, s is an integer of 0 to 3, and R^{X} is a hydrogen atom or a methyl group.

Examples of the group having a hydroxy group include, but not particularly limited to, a group represented by the following formula (X-5): 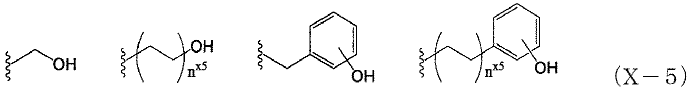 wherein n^{x5} is an integer of 1 to 5.

Examples of the group having a glycidyl group include, but not particularly limited to, a group represented by the following formula (X-6): 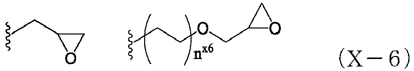 wherein n^{x6} is an integer of 1 to 5.

Examples of the group having a vinyl containing phenylmethyl group include, but not particularly limited to, a group represented by the following formula (X-7). 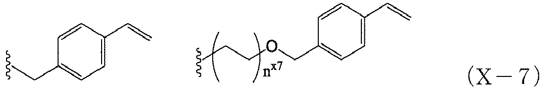 wherein n^{x7} is an integer of 1 to 5.

Examples of the group having a styrene group include, but not particularly limited to, a group represented by the following formula (X-8): 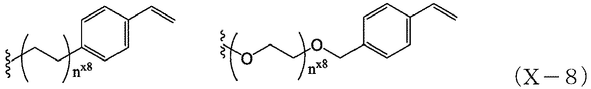 wherein n^{x8} is an integer of 1 to 5.

Examples of the group having various alkynyl groups include, but not particularly limited to, a group represented by the following formula (X-9): 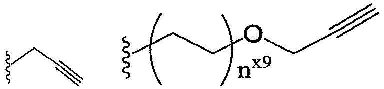 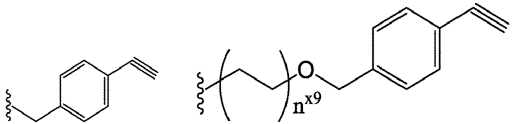 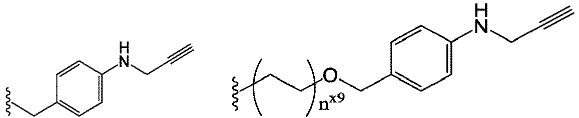 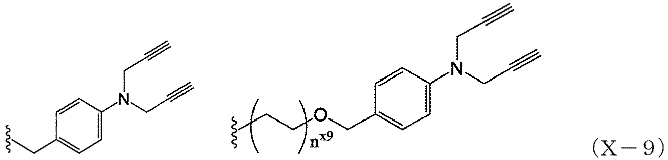 wherein n^{x9} is an integer of 1 to 5.

Examples of the group having a carbon-carbon double bond include a (meth)acryloyl group, a substituted or unsubstituted vinyl phenyl group, and a group represented by the following formula (X-10-1).

Examples of the group having a carbon-carbon triple bond include a substituted or unsubstituted ethynyl group, a substituted or unsubstituted propargyl group, and a group represented by the following formula (X-10-2) or (X-10-3) : 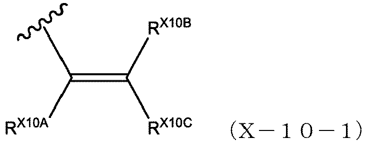 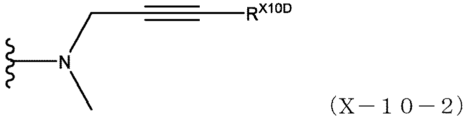 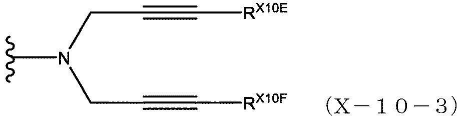 wherein R^{X10A}, R^{X10B}, and R^{X10C} are each independently a hydrogen atom or a monovalent hydrocarbon group having 1 to 20 carbon atoms, and wherein R^{X10D}, R^{X10E}, and R^{X10F} are each independently a hydrogen atom or a monovalent hydrocarbon group having 1 to 20 carbon atoms.

Among the above, from the viewpoint of ultraviolet curability, the crosslinkable group is preferably a (meth)acryloyl group, an epoxy (meth)acryloyl group, a urethane (meth)acryloyl group, a group having a glycidyl group, or a group containing a styrene group, more preferably a (meth)acryloyl group, an epoxy (meth)acryloyl group, or a group having a urethane (meth)acryloyl group, and still more preferably a group having a (meth)acryloyl group.

Here, the "dissociation group" refers to a group that is dissociated in the presence of a catalyst or without a catalyst. Among the dissociation groups, the acid dissociation group refers to a characteristic group that is cleaved in the presence of an acid to cause a change in an alkali soluble group or the like. Examples of the alkali soluble group include, but not particularly limited to, a phenolic hydroxy group, a carboxyl group, a sulfonic acid group, and a hexafluoroisopropanol group. Among them, a phenolic hydroxy group and a carboxyl group are preferable, and a phenolic hydroxy group is particularly preferable, from the viewpoint of the easy availability of an introduction reagent. The acid dissociation group preferably has the property of causing chained cleavage reaction in the presence of an acid, for achieving pattern formation with high sensitivity and high resolution. The acid dissociation group is not particularly limited, but can be arbitrarily selected for use from among, for example, those proposed in hydroxystyrene resins, (meth)acrylic acid resins, and the like for use in chemically amplified resist compositions for KrF or ArF.

Specific examples of the acid dissociation group include those described in International Publication No. WO 2016/158168. Preferable examples of the acid dissociation group include a group selected from the group consisting of a 1-substituted ethyl group, a 1-substituted n-propyl group, a 1-branched alkyl group, a silyl group, an acyl group, a 1-substituted alkoxymethyl group, a cyclic ether group, an alkoxycarbonyl group, and an alkoxycarbonylalkyl group, which has the property of being dissociated by an acid.

When the composition of the present embodiment is a positive type, at least any of the base material (A) and the polyphenol compound (B) has a dissociation group, or an additive agent that causes a change in the solubility in a developing solution due to the effect of exposure can be used in combination. Similarly, when the composition of the present embodiment is a negative type, a separate crosslinking agent may be used in combination, or at least any of the base material (A) and the polyphenol compound (B) may have a crosslinkable group.

### (Compound represented by formula (1))

In the present embodiment, as the polyphenol compound (B), a compound represented by the following formula (1) can be used. The compound represented by the formula (1) has high heat resistance attributed to its rigid structure, in spite of its relatively low molecular weight, and can therefore be used even under high temperature baking conditions. Also, the compound represented by the formula (1) has tertiary carbon or quaternary carbon in the molecule, which inhibits crystallinity, and is thus suitably used as a composition for lithography or a resist composition that is used in production of a film for lithography or a film for resist.

In the present embodiment, the molecular weight of the compound represented by the formula (1) is preferably 200 to 9900, more preferably 200 to 4900, and still more preferably 200 to 2900 from the viewpoints of reducing defects in a film to be formed by using the composition containing the compound and of a good pattern shape. As the weight average molecular weight, a value obtained by measuring the weight average molecular weight in terms of polystyrene, using GPC, can be used.

In addition, the compound represented by the following formula (1) has high solubility in a safe solvent and has good film-forming properties and sensitivity. Therefore, when a composition containing such a compound is used to form a film, a resist pattern with a good shape can be obtained. Furthermore, the compound represented by the formula (1) is a compound that has a relatively high carbon concentration, and can therefore also impart high etching resistance to a film to be obtained by using this compound. 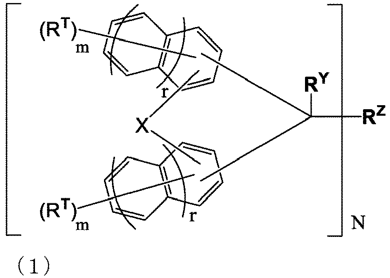
wherein R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms and optionally having a substituent, or a single bond;
each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
R^{z} contains at least two iodine atoms;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2); and
N is an integer of 1 to 4 (provided that when N is an integer of 2 or larger, N structural formulas within the parentheses [ ] are the same or different).

In the formula (1), with respect to R^{Y}, example of the alkyl group having 1 to 30 carbon atoms and optionally having a substituent include, but not particularly limited to, an unsubstituted methyl group, ethyl group, n-propyl group, i-propyl group, n-butyl group, i-butyl group, t-butyl group, cyclopropyl group, and cyclobutyl group, and examples thereof further include a methyl group, an ethyl group, a n-propyl group, an i-propyl group, a n-butyl group, an i-butyl group, a t-butyl group, a cyclopropyl group, and a cyclobutyl group, having a substituent such as a halogen atom, a nitro group, an amino group, a thiol group, a hydroxy group or a group in which a hydrogen atom of a hydroxy group is replaced with an acid dissociation group.

Examples of the aryl group having 6 to 30 carbon atoms and optionally having a substituent include an unsubstituted phenyl group, naphthalene group, and biphenyl group, and examples thereof further include a phenyl group, a naphthalene group, and a biphenyl group, having a substituent such as a halogen atom, a nitro group, an amino group, a thiol group, a hydroxy group, or a group in which a hydrogen atom of a hydroxy group is replaced with an acid dissociation group.

In the formula (1), with respect to R^{Z}, the N-valent group having 1 to 60 carbon atoms and optionally having a substituent refers to an alkyl group having 1 to 60 carbon atoms and optionally having a substituent when N is 1, an alkylene group having 1 to 60 carbon atoms when N is 2, an alkanetriyl group having 1 to 60 carbon atoms when N is 3, and an alkanetetrayl group having 1 to 60 carbon atoms when N is 4. Examples of the N-valent group include groups having linear hydrocarbon groups, branched hydrocarbon groups, and alicyclic hydrocarbon groups. Here, the alicyclic hydrocarbon group also includes a bridged alicyclic hydrocarbon group. Also, the N-valent group may have an aromatic group having 6 to 60 carbon atoms.

Also, the N-valent group may have an alicyclic hydrocarbon group, a double bond, a heteroatom, or an aromatic group having 6 to 60 carbon atoms. Here, the alicyclic hydrocarbon group also includes a bridged alicyclic hydrocarbon group.

The N-valent group having 1 to 60 carbon atoms and optionally having a substituent may be a group having 6 to 60 carbon atoms and optionally having a substituent, and examples thereof include an unsubstituted phenyl group, naphthalene group, biphenyl group, anthracyl group, pyrenyl group, cyclohexyl group, cyclododecyl group, dicyclopentyl group, tricyclodecyl group, adamantyl group, phenylene group, naphthalenediyl group, biphenyldiyl group, anthracenediyl group, pyrenediyl group, cyclohexanediyl group, cyclododecanediyl group, dicyclopentanediyl group, tricyclodecanediyl group, adamantanediyl group, benzenetriyl group, naphthalenetriyl group, biphenyltriyl group, anthracenetriyl group, pyrenetriyl group, cyclohexanetriyl group, cyclododecanetriyl group, dicyclopentanetriyl group, tricyclodecanetriyl group, adamantanetriyl group, benzenetetrayl group, naphthalenetetrayl group, biphenyltetrayl group, anthracenetetrayl group, pyrenetetrayl group, cyclohexanetetrayl group, cyclododecanetetrayl group, dicyclopentanetetrayl group, tricyclodecanetetrayl group, and adamantanetetrayl group. Examples thereof further include a phenyl group, a naphthalene group, a biphenyl group, an anthracyl group, a pyrenyl group, a cyclohexyl group, a cyclododecyl group, a dicyclopentyl group, a tricyclodecyl group, an adamantyl group, a phenylene group, a naphthalenediyl group, a biphenyldiyl group, an anthracenediyl group, a pyrenediyl group, a cyclohexanediyl group, a cyclododecanediyl group, a dicyclopentanediyl group, a tricyclodecanediyl group, an adamantanediyl group, a benzenetriyl group, a naphthalenetriyl group, a biphenyltriyl group, an anthracenetriyl group, a pyrenetriyl group, a cyclohexanetriyl group, a cyclododecanetriyl group, a dicyclopentanetriyl group, a tricyclodecanetriyl group, an adamantanetriyl group, a benzenetetrayl group, a naphthalenetetrayl group, a biphenyltetrayl group, an anthracenetetrayl group, a pyrenetetrayl group, a cyclohexanetetrayl group, a cyclododecanetetrayl group, a dicyclopentanetetrayl group, a tricyclodecanetetrayl group, and an adamantanetetrayl group, having a substituent such as a halogen atom, a nitro group, an amino group, a thiol group, a hydroxy group or a group in which a hydrogen atom of a hydroxy group is replaced with an acid dissociation group.

In the formula (1), with respect to R^{T}, example of the alkyl group having 1 to 30 carbon atoms and optionally having a substituent include, but not particularly limited to, an unsubstituted methyl group, ethyl group, n-propyl group, i-propyl group, n-butyl group, i-butyl group, t-butyl group, cyclopropyl group, and cyclobutyl group, and examples thereof further include a methyl group, an ethyl group, a n-propyl group, an i-propyl group, a n-butyl group, an i-butyl group, a t-butyl group, a cyclopropyl group, and a cyclobutyl group, having a substituent such as a halogen atom, a nitro group, an amino group, a thiol group, a hydroxy group or a group in which a hydrogen atom of a hydroxy group is replaced with an acid dissociation group.

Examples of the aryl group having 6 to 30 carbon atoms and optionally having a substituent include, but not particularly limited to, an unsubstituted phenyl group, naphthalene group, and biphenyl group, and examples thereof further include a phenyl group, a naphthalene group, and a biphenyl group, having a substituent such as a halogen atom, a nitro group, an amino group, a thiol group, a hydroxy group, or a group in which a hydrogen atom of a hydroxy group is replaced with an acid dissociation group.

Examples of the alkenyl group having 2 to 30 carbon atoms and optionally having a substituent thereof include, but not particularly limited to, an unsubstituted propenyl group and butenyl group, and examples thereof further include a propenyl group and a butenyl group, having a substituent such as a halogen atom, a nitro group, an amino group, a thiol group, a hydroxy group, or a group in which a hydrogen atom of a hydroxy group is replaced with an acid dissociation group.

Examples of the alkynyl group having 2 to 30 carbon atoms and optionally having a substituent thereof include, but not particularly limited to, an ethynyl group and a propagyl group, and examples thereof further include an ethynyl group and a propagyl group, having a substituent such as a halogen atom, a nitro group, an amino group, a thiol group, a hydroxy group, or a group in which a hydrogen atom of a hydroxy group is replaced with an acid dissociation group.

Examples of the alkoxy group having 1 to 30 carbon atoms and optionally having a substituent include, but not particularly limited to, an unsubstituted methoxy group, ethoxy group, propoxy group, cyclohexyloxy group, phenoxy group, naphthalenoxy group, and biphenyl group, and examples thereof further include a methoxy group, an ethoxy group, a propoxy group, a cyclohexyloxy group, a phenoxy group and a naphthalenoxy group, having a substituent such as a halogen atom, a nitro group, an amino group, a thiol group, a hydroxy group, or a group in which a hydrogen atom of a hydroxy group is replaced with an acid dissociation group.

Examples of the crosslinkable group and the dissociation group include those mentioned above.

In the formula (1), when r is 0, the cyclic structure shown in the parentheses is a benzene ring, and when r is 1, the cyclic structure shown in the parentheses is a naphthalene ring. When r is 2, the cyclic structure shown in the parentheses is a tricyclic structure such as an anthracene ring and a phenanthrene ring.

### (Compound represented by formula (1A))

In the present embodiment, from the viewpoint of easy availability, the compound represented by the formula (1) is preferably a compound represented by the following formula (1A): 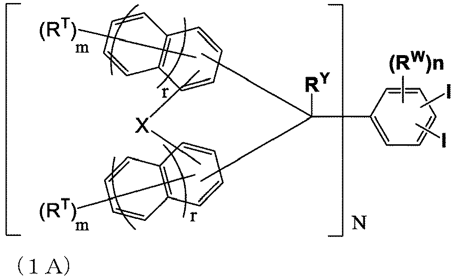
wherein R^{Y} is a hydrogen atom, an alkyl group having 1 to 30 carbon atoms and optionally having a substituent, or an aryl group having 6 to 30 carbon atoms and optionally having a substituent;
R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2);
each n is independently an integer of 0 to 3; and
N is an integer of 1 to 4 (provided that when N is an integer of 2 or larger, N structural formulas within the parentheses [ ] are the same or different).

Specific examples of the compound represented by the above formula (1) will be listed below, though the compound represented by the above formula (1) is not limited thereto:

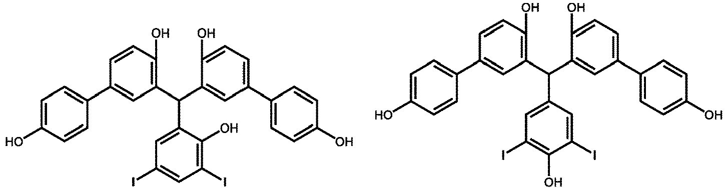

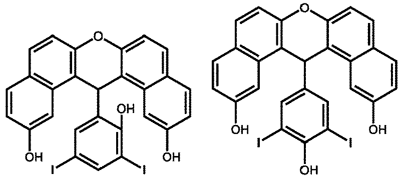

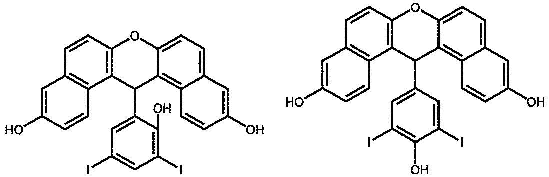

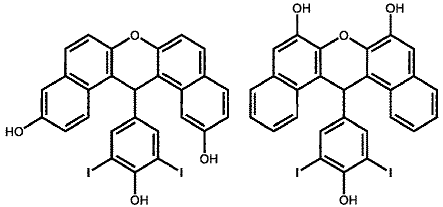

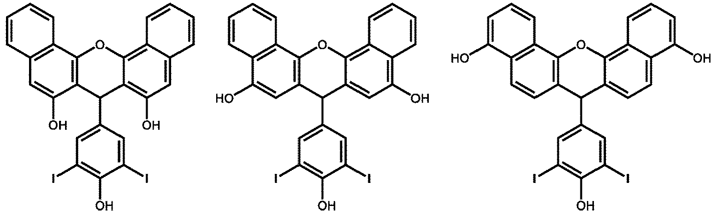

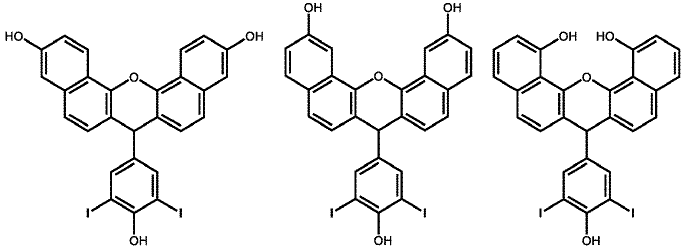

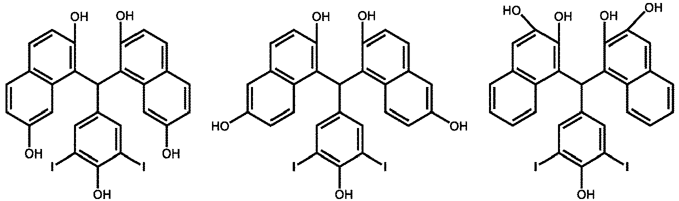

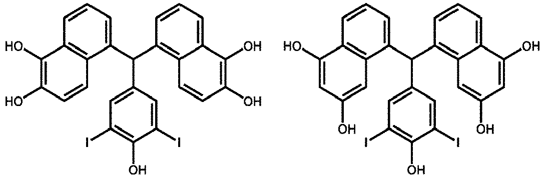

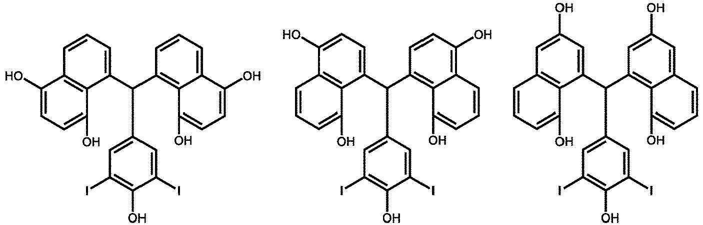

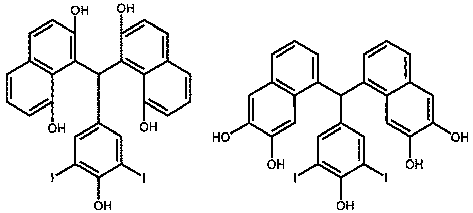

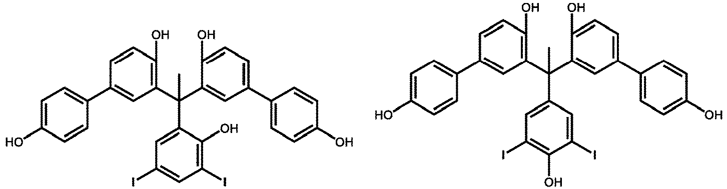

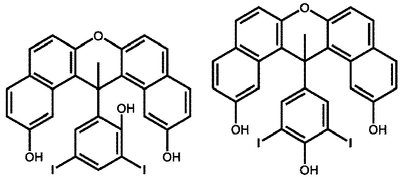

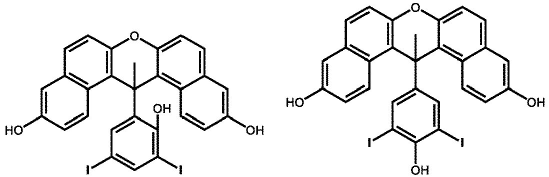

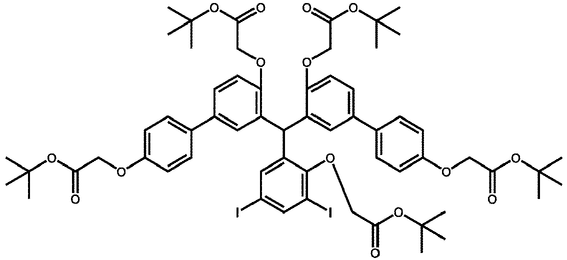

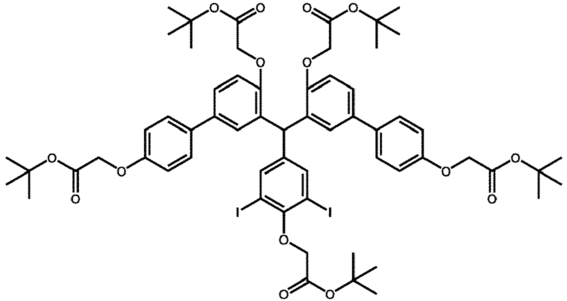

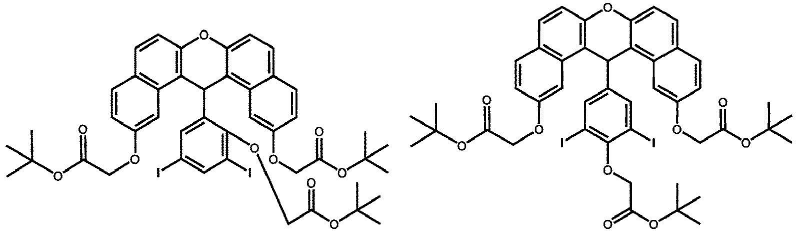

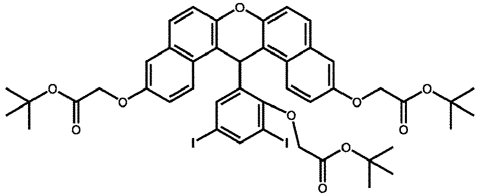

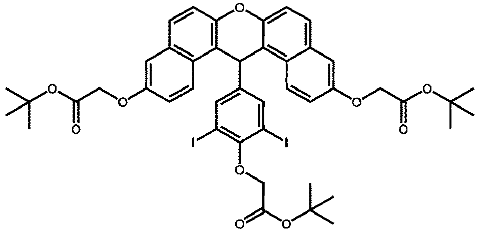

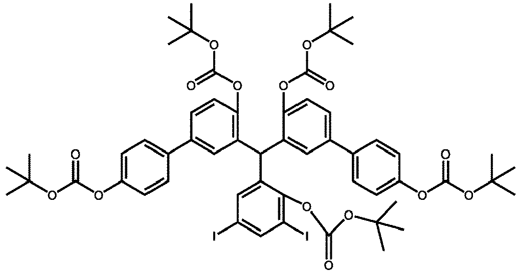

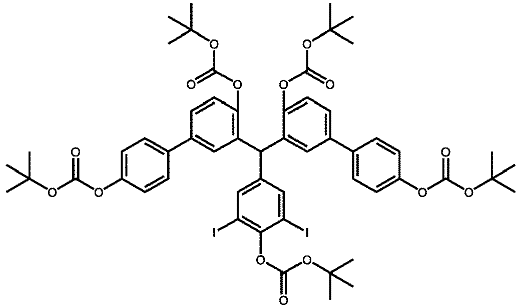

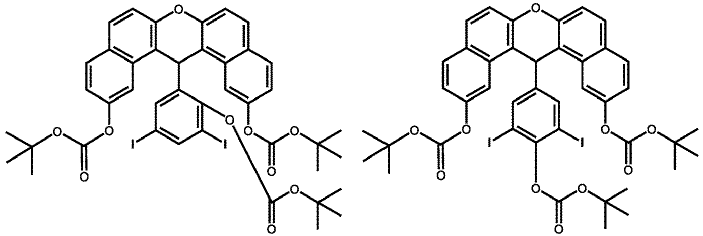

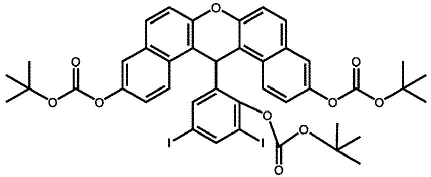

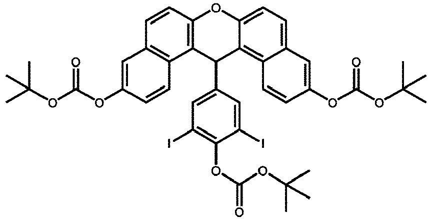

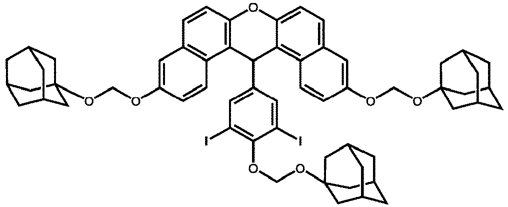

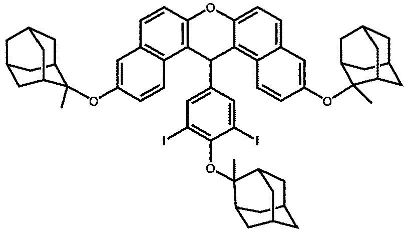

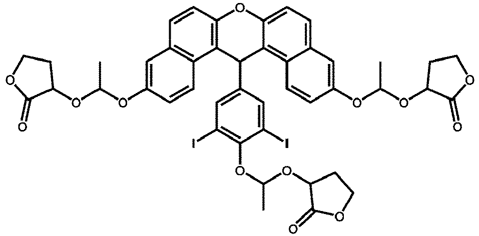

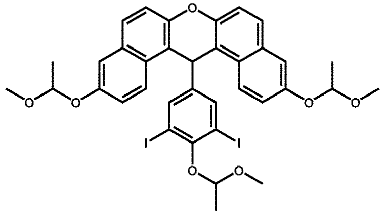

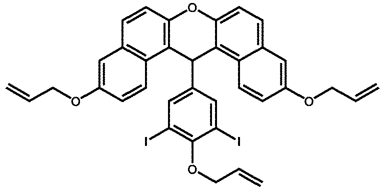

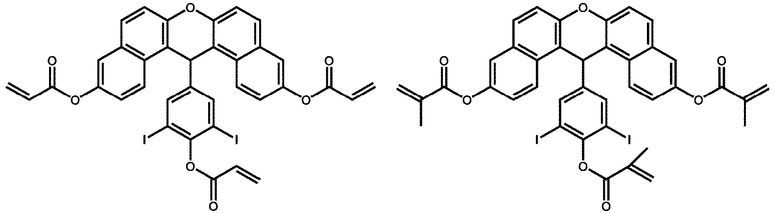

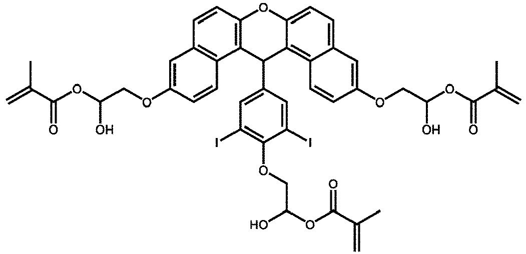

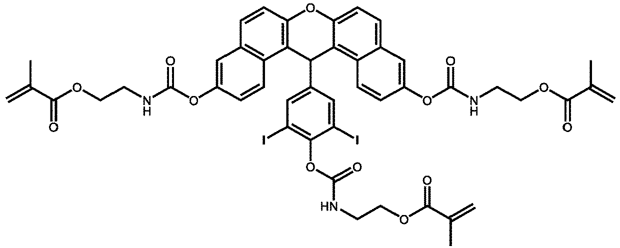

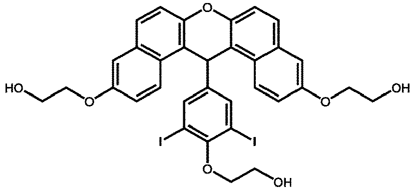

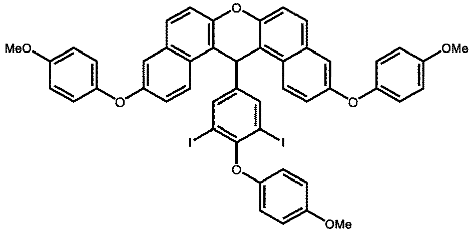

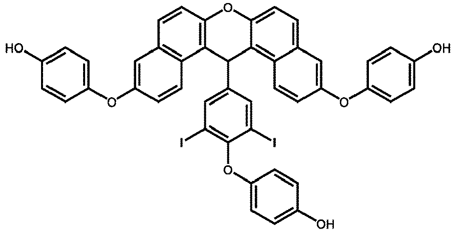

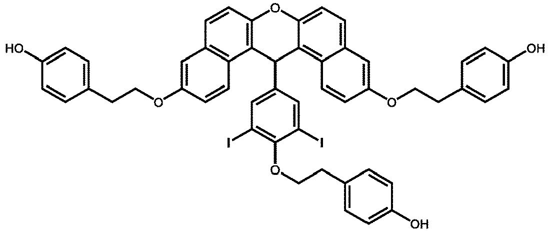

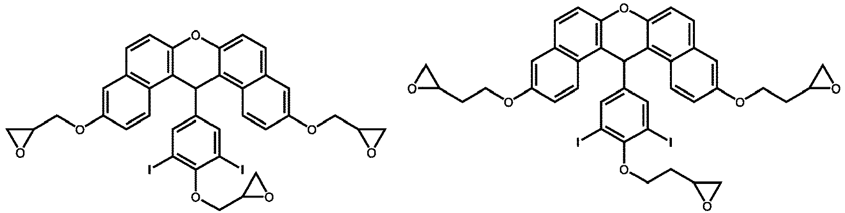

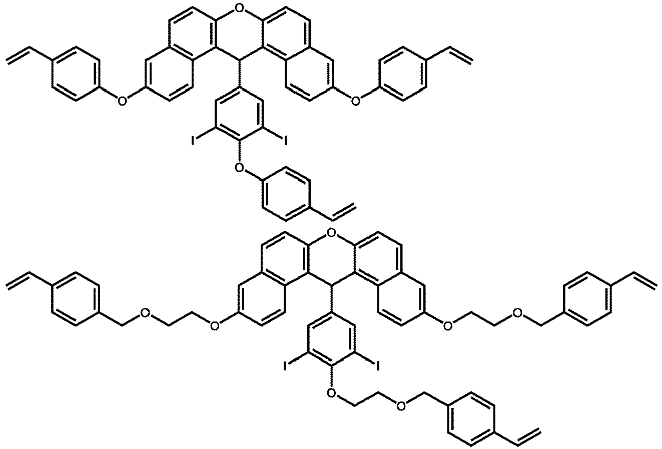

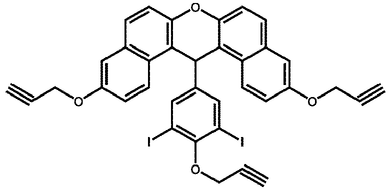

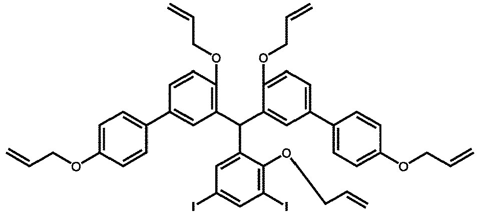

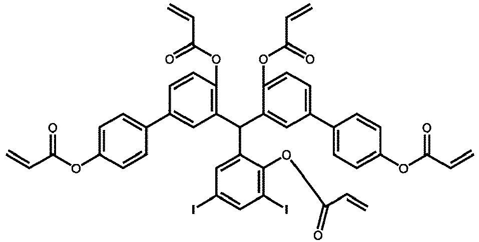

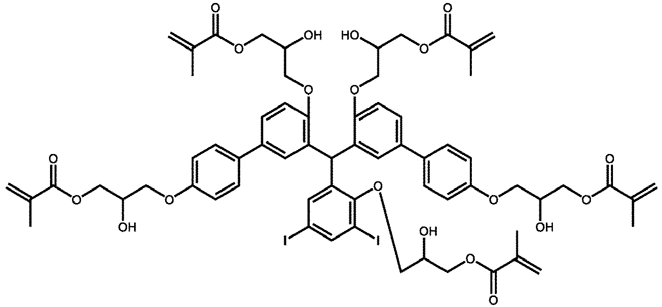

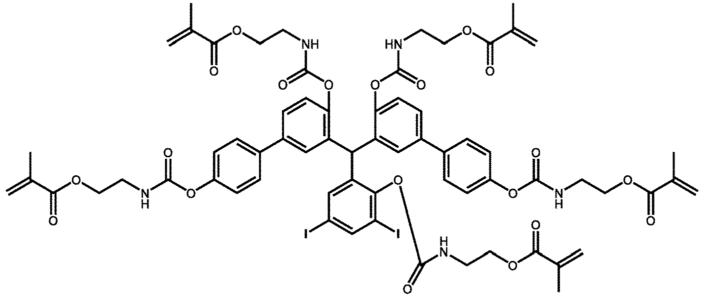

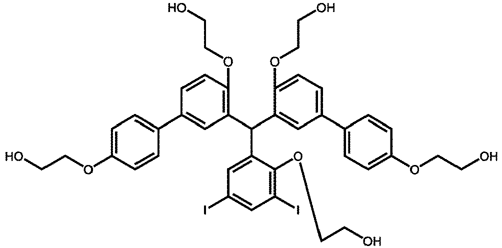

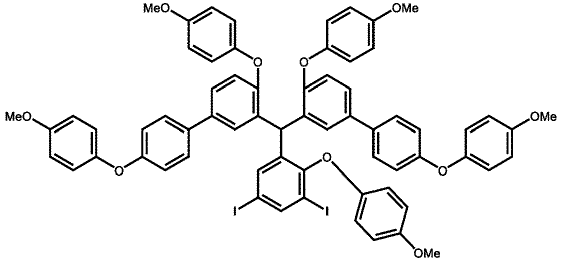

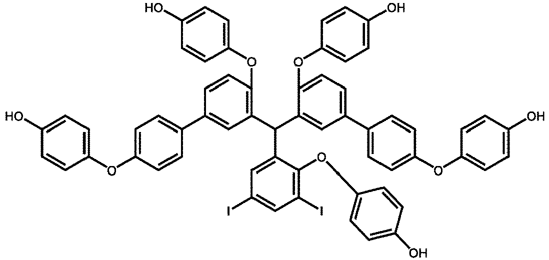

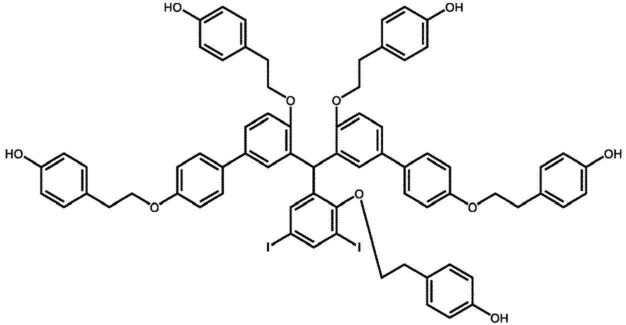

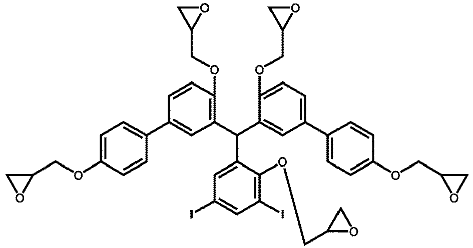

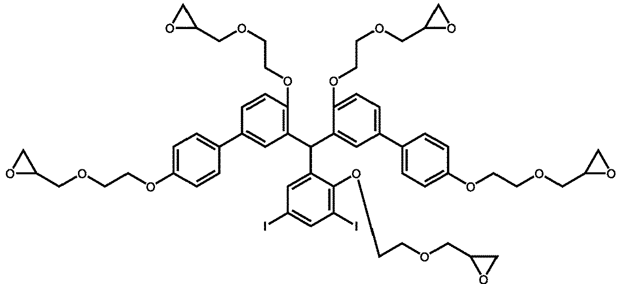

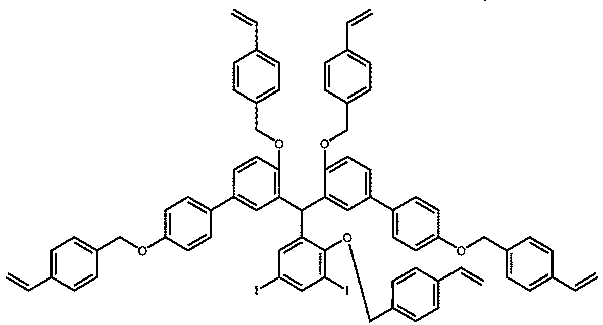

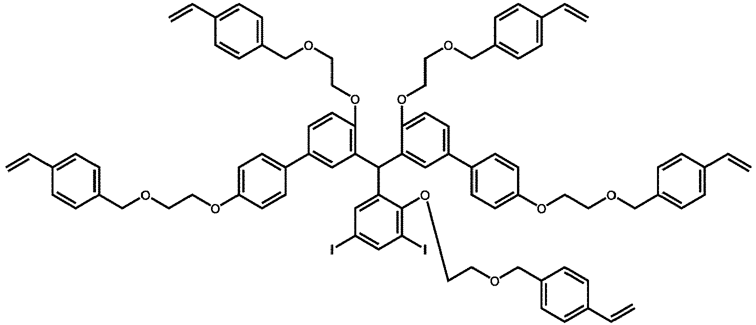

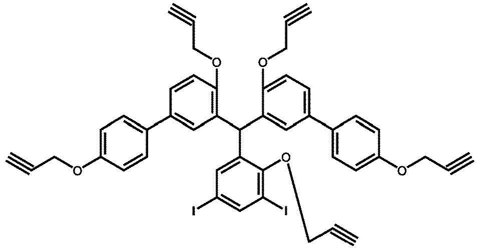

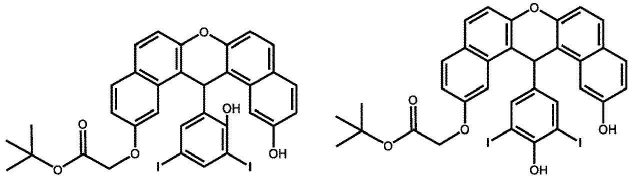

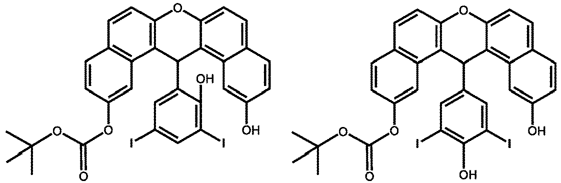

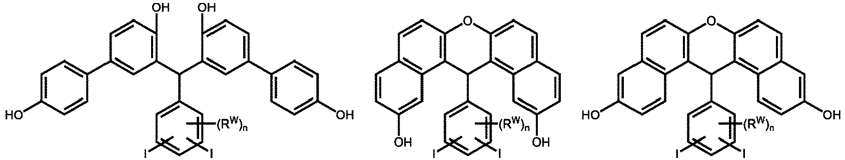

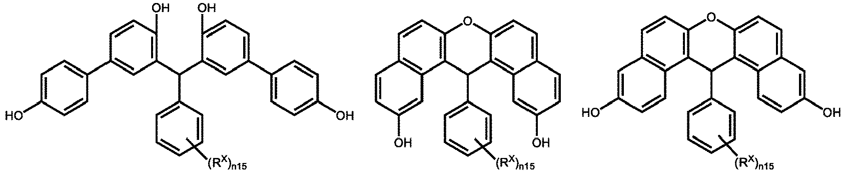

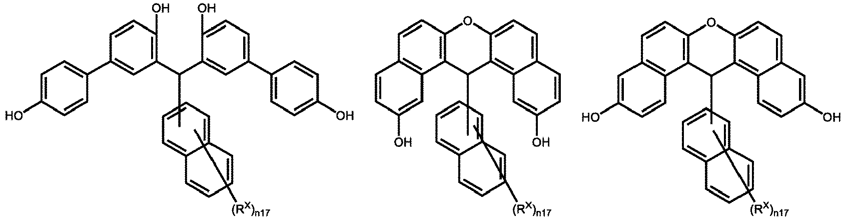

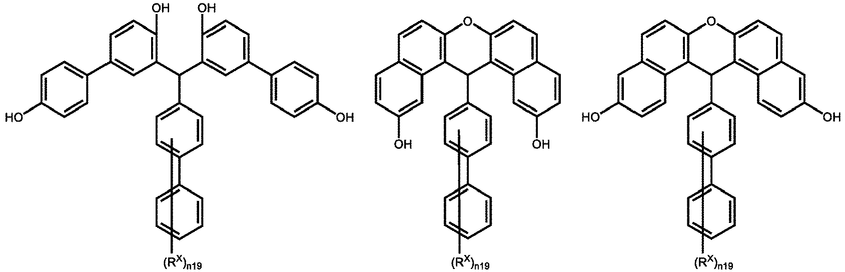

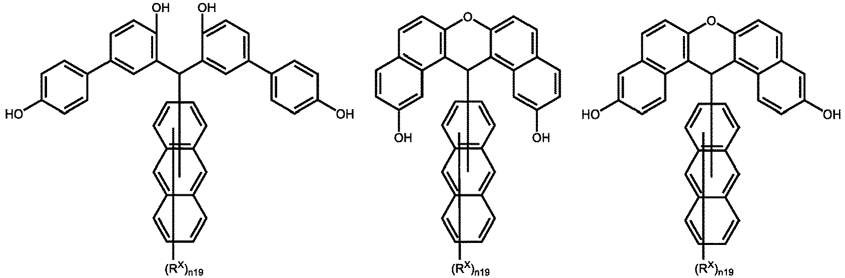

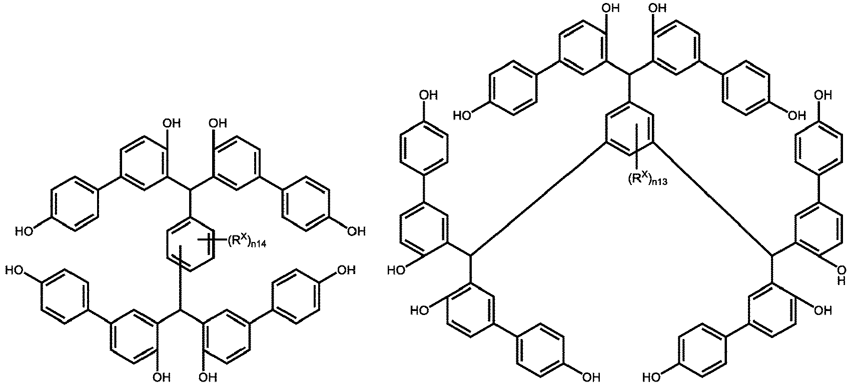

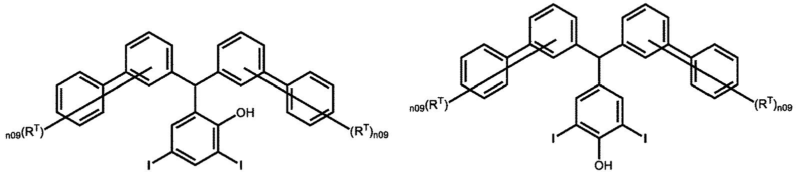

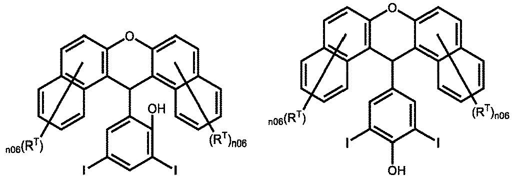

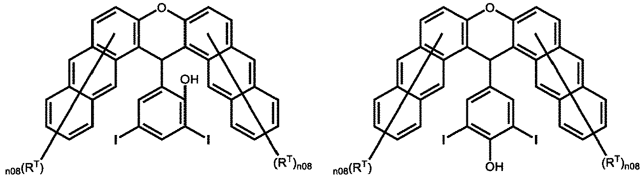

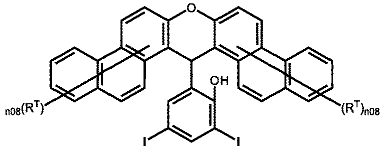

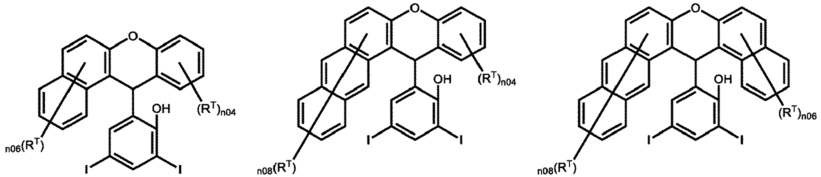

Here, R^{T} is the same as defined in the formula (1). R^{X} and R^{W} are the same as defined in the formula (1A). n is the same as defined in the formula (1A). However, the sum of the iodine atoms in all R^{X} in one molecule is two or more. Each n04 is independently an integer of 0 to 4. Each n06 is independently an integer of 0 to 6. Each n08 is independently an integer of 0 to 8. Each n09 is independently an integer of 0 to 9. Each n13 is independently an integer of 1 to 3. Each n14 is independently an integer of 1 to 4. Each n15 is independently an integer of 1 to 5. Each n17 is independently an integer of 1 to 7. Each n19 is independently an integer of 1 to 9. However, at least one of n04, n06, n08, and n09 in one molecule is one or more.

### (Resin having structure represented by formula (2))

The composition of the present embodiment may contain a resin having a structure represented by the following formula (2). The resin having a structure represented by the formula (2) is a resin obtained by using the compound represented by the formula (1) mentioned above as a monomer. Such a resin can be obtained, for example, by reacting the compound represented by the above formula (1) with a crosslinking compound. 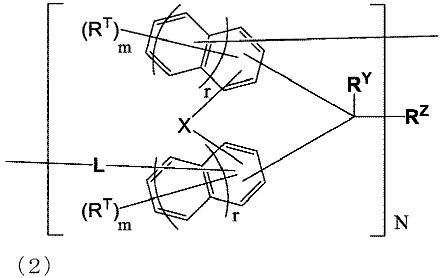
wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms and optionally having a substituent, or a single bond;
each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
R^{z} contains at least two iodine atoms;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2); and
N is an integer of 1 to 4 (provided that when N is an integer of 2 or larger, N structural formulas within the parentheses [ ] are the same or different).

In the formula (2), L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, and the alkylene group, the arylene group and the alkoxylene group each optionally contain an ether bond, a ketone bond or an ester bond. Each of the alkylene group and the alkoxylene group may be a linear, branched or cyclic group.

In the present embodiment, the weight average molecular weight of the resin having a structure represented by the formula (2) is preferably 300 to 10000, more preferably 300 to 5000, and still more preferably 300 to 3000 from the viewpoints of reducing defects in a film to be formed by using the composition and of a good pattern shape. As the weight average molecular weight, a value obtained by measuring the weight average molecular weight in terms of polystyrene, using GPC, can be used.

### (Resin represented by formula (2A))

In the present embodiment, the resin represented by the formula (2) is preferably a resin represented by the following formula (2A) from the viewpoint of easy availability. 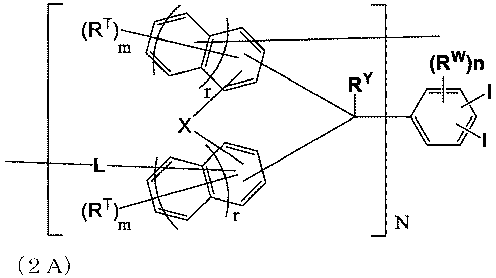
wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 30 carbon atoms and optionally having a substituent;
R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2);
each n is independently an integer of 0 to 3; and
N is an integer of 1 to 4 (provided that when N is an integer of 2 or larger, N structural formulas within the parentheses [ ] are the same or different).

### (Method for producing resin having structure represented by formula (2))

The resin having a structure represented by the formula (2) is obtained by reacting the compound represented by the above formula (1) with a crosslinking compound. As the crosslinking compound, a publicly known compound can be used without particular limitations as long as it can oligomerize or polymerize the compound represented by the above formula (1). Specific examples of the crosslinking compound include, but not particularly limited to, an aldehyde, a ketone, a carboxylic acid, a carboxylic acid halide, a halogen containing compound, an amino compound, an imino compound, an isocyanate, and an unsaturated hydrocarbon group containing compound.

Specific examples of the resin having a structure represented by the above formula (2) include a resin that is made novolac through, for example, a condensation reaction between the compound represented by the above formula (1) with an aldehyde and/or a ketone, which is a crosslinking compound.

The resin having a structure represented by the formula (2) can also be obtained upon the synthesis reaction for the compound represented by the above formula (1). For example, upon synthesizing the compound represented by the above formula (1), there are some cases that the compound represented by the above formula (1) is subjected to condensation reaction with an aldehyde or a ketone and the resin having a structure represented by the formula (2) is obtained.

### [Solvent (S)]

As a solvent according to the present embodiment, a publicly known solvent can be arbitrarily used as long as it can at least dissolve the polyphenol compound (B) mentioned above. Specific examples of the solvent can include, but not particularly limited to, an ethylene glycol monoalkyl ether acetate such as ethylene glycol monomethyl ether acetate, ethylene glycol monoethyl ether acetate, ethylene glycol mono-n-propyl ether acetate, and ethylene glycol mono-n-butyl ether acetate; an ethylene glycol monoalkyl ether such as ethylene glycol monomethyl ether and ethylene glycol monoethyl ether; a propylene glycol monoalkyl ether acetate such as propylene glycol monomethyl ether acetate (PGMEA), propylene glycol monoethyl ether acetate, propylene glycol mono-n-propyl ether acetate, and propylene glycol mono-n-butyl ether acetate; a propylene glycol monoalkyl ethers such as propylene glycol monomethyl ether (PGME) and propylene glycol monoethyl ether; a lactate ester such as methyl lactate, ethyl lactate, n-propyl lactate, n-butyl lactate, and n-amyl lactate; an aliphatic carboxylic acid ester such as methyl acetate, ethyl acetate, n-propyl acetate, n-butyl acetate, n-amyl acetate, n-hexyl acetate, methyl propionate, and ethyl propionate; another ester such as methyl 3-methoxypropionate, ethyl 3-methoxypropionate, methyl 3-ethoxypropionate, ethyl 3-ethoxypropionate, methyl 3-methoxy-2-methylpropionate, 3-methoxybutyl acetate, 3-methyl-3-methoxybutyl acetate, butyl 3-methoxy-3-methylpropionate, butyl 3-methoxy-3-methylbutyrate, methyl acetoacetate, methyl pyruvate, and ethyl pyruvate; an aromatic hydrocarbon such as toluene and xylene; a ketone such as acetone, 2-butanone, 2-heptanone, 3-heptanone, 4-heptanone, cyclopentanone (CPN), and cyclohexanone (CHN); an amide such as N,N-dimethylformamide, N-methylacetamide, N,N-dimethylacetamide, and N-methylpyrrolidone; and a lactone such as γ-lactone. The solvent used in the present embodiment is preferably a safe solvent, more preferably at least one selected from PGMEA, PGME, CHN, CPN, 2-heptanone, anisole, butyl acetate and ethyl lactate, and still more preferably at least one selected from PGMEA, PGME, CHN, CPN and ethyl lactate.

In the present embodiment, the amount of the solid component and the amount of the solvent are not particularly limited, but preferably the solid component is 1 to 80% by mass and the solvent is 20 to 99% by mass, more preferably the solid component is 1 to 50% by mass and the solvent is 50 to 99% by mass, still more preferably the solid component is 2 to 40% by mass and the solvent is 60 to 98% by mass, and particularly preferably the solid component is 2 to 10% by mass and the solvent is 90 to 98% by mass, based on the total mass of the amount of the solid component and the solvent.

### [Acid generating agent (C)]

The composition of the present embodiment preferably contains one or more acid generating agents (C) generating an acid directly or indirectly by irradiation of any radiation selected from visible light, ultraviolet, excimer laser, electron beam, extreme ultraviolet (EUV), X-ray and ion beam. The acid generating agent (C) is not particularly limited, and, for example, an acid generating agent described in International Publication No. WO 2013/024778 can be used. The acid generating agent (C) can be used alone or in combination of two or more kinds.

The amount of the acid generating agent (C) used is preferably 0.001 to 49% by mass of the total mass of the solid component, more preferably 1 to 40% by mass, still more preferably 3 to 30% by mass, and particularly preferably 10 to 25% by mass. By using the acid generating agent (C) within the above range, a pattern profile with high sensitivity and low edge roughness tends to be obtained. In the present embodiment, the acid generation method is not particularly limited as long as an acid is generated in the system. By using excimer laser instead of ultraviolet such as g-ray and i-ray, finer processing is possible, and also by using electron beam, extreme ultraviolet, X-ray or ion beam as a high energy ray, further finer processing is possible.

### [Crosslinking agent (G)]

In the present embodiment, one or more crosslinking agents (G) can be contained in the composition. The crosslinking agent (G) refers to a compound that can at least crosslink either base material (A) or polyphenol compound (B). It is preferable that the crosslinking agent (G) be an acid crosslinking agent capable of intramolecularly or intermolecularly crosslinking the base material (A) in the presence of an acid generated from the acid generating agent (C). Examples of such an acid crosslinking agent can include a compound having one or more groups capable of crosslinking the base material (A) (hereinafter, referred to as a "crosslinkable group").

Examples of the crosslinkable group can include: (i) a hydroxyalkyl group or a group derived therefrom, such as a hydroxy (alkyl group having 1 to 6 carbon atoms), alkoxy having 1 to 6 carbon atoms (alkyl group having 1 to 6 carbon atoms) and acetoxy (alkyl group having 1 to 6 carbon atoms); (ii) a carbonyl group or a group derived therefrom, such as a formyl group and a carboxy (alkyl group having 1 to 6 carbon atoms); (iii) a nitrogenous group containing group such as a dimethylaminomethyl group, a diethylaminomethyl group, a dimethylolaminomethyl group, a diethylolaminomethyl group and a morpholinomethyl group; (iv) a glycidyl group containing group such as a glycidyl ether group, a glycidyl ester group and a glycidylamino group; (v) a group derived from an aromatic group such as an allyloxy having 1 to 6 carbon atoms (alkyl group having 1 to 6 carbon atoms) and an aralkyloxy having 1 to 6 carbon atoms (alkyl group having 1 to 6 carbon atoms) such as a benzyloxymethyl group and a benzoyloxymethyl group; and (vi) a polymerizable multiple bond containing group such as a vinyl group and an isopropenyl group. As the crosslinkable group of the crosslinking agent (G) according to the present embodiment, a hydroxyalkyl group, an alkoxyalkyl group and the like are preferable, and an alkoxymethyl group is particularly preferable.

The crosslinking agent (G) having the crosslinkable group is not particularly limited, and, for example, an acid crosslinking agent described in International Publication No. WO 2013/024778 can be used. The crosslinking agent (G) can be used alone or in combination of two or more kinds.

In the present embodiment, the amount of the crosslinking agent (G) used is preferably 0.5 to 50% by mass of the total mass of the solid component, more preferably 0.5 to 40% by mass, still more preferably 1 to 30% by mass, and particularly preferably 2 to 20% by mass. When the content ratio of the crosslinking agent (G) is 0.5% by mass or more, there is a tendency that the inhibiting effect of the solubility of a resist film in an alkaline developing solution is improved and that a decrease in the film remaining rate, as well as occurrence of swelling and meandering of a pattern, can be inhibited. On the other hand, when the amount is 50% by mass or less, there is a tendency that a decrease in heat resistance as a resist can be inhibited.

### [Acid diffusion controlling agent (E)]

In the present embodiment, the composition of the present embodiment may contain an acid diffusion controlling agent (E) having a function of controlling diffusion of an acid generated from an acid generating agent by radiation irradiation in a resist film to inhibit any unpreferable chemical reaction in an unexposed region or the like. The use of the acid diffusion controlling agent (E) tends to improve the storage stability of the composition of the present embodiment. Also, by using the acid diffusion controlling agent (E), not only the resolution of a film formed by using the composition of the present embodiment can be improved, but the line width change of a resist pattern due to variation in the post exposure delay time before radiation irradiation and the post exposure delay time after radiation irradiation can also be inhibited, so that the process stability tends to be excellent. Examples of the acid diffusion controlling agent (E) include, but not particularly limited to, a radiation degradable basic compound such as a nitrogen atom containing basic compound, a basic sulfonium compound, and a basic iodonium compound.

The acid diffusion controlling agent (E) is not particularly limited, and, for example, an acid diffusion controlling agent described in International Publication No. WO 2013/024778 can be used. The acid diffusion controlling agent (E) can be used alone or in combination of two or more kinds.

The content of the acid diffusion controlling agent (E) is preferably 0.001 to 49% by mass of the total mass of the solid component, more preferably 0.01 to 10% by mass, still more preferably 0.01 to 5% by mass, and particularly preferably 0.01 to 3% by mass. When the content of the acid diffusion controlling agent (E) is within the range, there is a tendency that a decrease in resolution, and deterioration of the pattern shape and the dimension fidelity or the like can be prevented. Moreover, even though the post exposure delay time from electron beam irradiation to heating after radiation irradiation becomes longer, deterioration of the shape of the pattern upper layer portion can be inhibited. Also, when the content is 10% by mass or less, there is a tendency that a decrease in sensitivity, and developability of the unexposed portion or the like can be prevented. Also, by using such an acid diffusion controlling agent, the storage stability of a resist composition is improved, also along with improvement of the resolution, the line width change of a resist pattern due to variation in the post exposure delay time before radiation irradiation and the post exposure delay time after radiation irradiation can be inhibited, so that the process stability tends to be extremely excellent.

### [Further component (F)]

To the composition of the present embodiment, if required, as the further component (F), one kind or two kinds or more of various additive agents such as a dissolution promoting agent, a dissolution controlling agent, a sensitizing agent, a surfactant, and an organic carboxylic acid or an oxo acid of phosphor or derivative thereof can be added.

### (Dissolution promoting agent)

The dissolution promoting agent is a component having a function of, when the solubility of a solid component is too low, increasing the solubility of the solid component in a developing solution to moderately increase the dissolution rate of that compound upon developing. As the dissolution promoting agent, those having a low molecular weight are preferable, and examples thereof can include a phenolic compound having a low molecular weight. Examples of the phenolic compound having a low molecular weight can include a bisphenol and a tris(hydroxyphenyl)methane. These dissolution promoting agents can be used alone or in mixture of two or more kinds.

The content of the dissolution promoting agent, which is arbitrarily adjusted according to the kind of the solid component to be used, is preferably 0 to 49% by mass of the total mass of the solid component, more preferably 0 to 5% by mass, still more preferably 0 to 1% by mass, and particularly preferably 0% by mass.

### (Dissolution controlling agent)

The dissolution controlling agent is a component having a function of, when the solubility of a solid component is too high, controlling the solubility of the solid component in a developing solution to moderately decrease the dissolution rate upon developing. As such a dissolution controlling agent, the one which does not chemically change in steps such as calcination of resist coating, radiation irradiation, and development is preferable.

The dissolution controlling agent is not particularly limited, and examples thereof can include an aromatic hydrocarbon such as phenanthrene, anthracene and acenaphthene; a ketone such as acetophenone, benzophenone and phenyl naphthyl ketone; and a sulfone such as methyl phenyl sulfone, diphenyl sulfone and dinaphthyl sulfone. These dissolution controlling agents can be used alone or in combination of two or more kinds.

The content of the dissolution controlling agent, which is arbitrarily adjusted according to the kind of the compound to be used, is preferably 0 to 49% by mass of the total mass of the solid component, more preferably 0 to 5% by mass, still more preferably 0 to 1% by mass, and particularly preferably 0% by mass.

### (Sensitizing agent)

The sensitizing agent is a component having a function of absorbing irradiated radiation energy, transmitting the energy to the acid generating agent (C), and thereby increasing the acid production amount, and improving the apparent sensitivity of a resist. Examples of such a sensitizing agent can include, but not particularly limited to, a benzophenone, a biacetyl, a pyrene, a phenothiazine and a fluorene. These sensitizing agents can be used alone or in combination of two or more kinds.

The content of the sensitizing agent, which is arbitrarily adjusted according to the kind of the compound to be used, is preferably 0 to 49% by mass of the total mass of the solid component, more preferably 0 to 5% by mass, still more preferably 0 to 1% by mass, and particularly preferably 0% by mass.

### (Surfactant)

The surfactant is a component having a function of improving coatability and striation of the composition of the present embodiment, and developability of a resist or the like. The surfactant may be any of anionic, cationic, nonionic, and amphoteric surfactants. Preferable examples of the surfactant include a nonionic surfactant. The nonionic surfactant has a good affinity with a solvent to be used in production of the composition of the present embodiment, and can further enhance the effects of the composition of the present embodiment. Examples of the nonionic surfactant include, but not particularly limited to, a polyoxyethylene higher alkyl ether, a polyoxyethylene higher alkyl phenyl ether, and a higher fatty acid diester of polyethylene glycol. Examples of commercially available products of these surfactants can include, hereinafter by trade name, EFTOP (manufactured by Jemco Inc.), MEGAFAC (manufactured by DIC Corporation), Fluorad (manufactured by Sumitomo 3M Limited), AsahiGuard, Surflon (hereinbefore, manufactured by Asahi Glass Co., Ltd.), Pepole (manufactured by Toho Chemical Industry Co., Ltd.), KP (manufactured by Shin-Etsu Chemical Co., Ltd.), and Polyflow (manufactured by Kyoeisha Chemical Co., Ltd.).

The content of the surfactant, which is arbitrarily adjusted according to the kind of the solid component to be used, is preferably 0 to 49% by mass of the total mass of the solid component, more preferably 0 to 5% by mass, still more preferably 0 to 1% by mass, and particularly preferably 0% by mass.

(Organic carboxylic acid or oxo acid of phosphor or derivative thereof)

For the purpose of prevention of sensitivity deterioration or improvement of a resist pattern shape and post exposure delay stability or the like, and as an additional optional component, the composition of the present embodiment can contain an organic carboxylic acid or an oxo acid of phosphor or derivative thereof. The organic carboxylic acid or the oxo acid of phosphor or derivative thereof can be used in combination with the acid diffusion controlling agent, or may be used alone. Suitable examples of the organic carboxylic acid include malonic acid, citric acid, malic acid, succinic acid, benzoic acid and salicylic acid. Examples of the oxo acid of phosphor or derivative thereof include phosphoric acid or derivative thereof such as ester including phosphoric acid, di-n-butyl phosphate and diphenyl phosphate; phosphonic acid or derivative thereof such as ester including phosphonic acid, dimethyl phosphonate, di-n-butyl phosphonate, phenylphosphonic acid, diphenyl phosphonate and dibenzyl phosphonate; and phosphinic acid and derivative thereof such as ester including phosphinic acid and phenylphosphinic acid. Among them, phosphonic acid is particularly preferable.

The organic carboxylic acid or the oxo acid of phosphor or derivative thereof can be used alone or in combination of two or more kinds. The content of the organic carboxylic acid or the oxo acid of phosphor or derivative thereof, which is arbitrarily adjusted according to the kind of the compound to be used, is preferably 0 to 49% by mass of the total mass of the solid component, more preferably 0 to 5% by mass, still more preferably 0 to 1% by mass, and particularly preferably 0% by mass.

### [Further additive agent]

Furthermore, the composition of the present embodiment can contain one kind or two kinds or more of additive agents other than the components mentioned above, if required. Examples of such an additive agent include a dye, a pigment and an adhesion aid. For example, when the composition contains a dye or a pigment, a latent image of the exposed portion is visualized and influence of halation upon exposure can be alleviated, which is preferable. Also, when the composition contains an adhesion aid, adhesiveness to a substrate can be improved, which is preferable. Furthermore, examples of the further additive agent can include a halation preventing agent, a storage stabilizing agent, a defoaming agent and a shape improving agent. Specific examples thereof can include 4-hydroxy-4'-methylchalkone.

In the composition of the present embodiment, the total content of the optional component (F) can be 0 to 99% by mass of the total mass of the solid component, and is preferably 0 to 49% by mass, more preferably 0 to 10% by mass, still more preferably 0 to 5% by mass, further preferably 0 to 1% by mass, and particularly preferably 0% by mass.

### [Content ratio of each component in composition]

In the composition of the present embodiment, the total amount of the base material (A) and the polyphenol compound (B) is preferably 50 to 99.4% by mass of the total mass of the solid component (summation of solid components including the base material (A), the polyphenol compound (B), and optionally used components such as acid generating agent (C), crosslinking agent (G), acid diffusion controlling agent (E), and further component (F), hereinafter the same), more preferably 55 to 90% by mass, still more preferably 60 to 80% by mass, and particularly preferably 60 to 70% by mass. When the total amount of the base material (A) and the polyphenol compound (B) is the above content, there is a tendency that resolution is further improved and that line edge roughness (LER) is further decreased. When the composition contains both the compound and the resin according to the present embodiment, the above content refers to an amount including both the compound and the resin according to the present embodiment.

In the composition of the present embodiment, the content ratio among the base material (A), the polyphenol compound (B), the acid generating agent (C), the crosslinking agent (G), the acid diffusion controlling agent (E) and an optional component (F) according to the present embodiment (base material (A)/polyphenol compound (B)/acid generating agent (C)/crosslinking agent (G)/acid diffusion controlling agent (E)/optional component (F)) is, based on the total mass of the solid component of the composition of the present embodiment,
preferably, 0 to 99.4% by mass/0.1 to 99.5% by mass/0.001 to 49% by mass/0.5 to 49% by mass/0.001 to 49% by mass/0 to 49% by mass;
more preferably, 0 to 97.5% by mass/1 to 99% by mass/1 to 40% by mass/0.5 to 40% by mass/0.01 to 10% by mass/0 to 5% by mass;
still more preferably, 0 to 95% by mass/1 to 50% by mass/3 to 30% by mass/1 to 30% by mass/0.01 to 5% by mass/0 to 1% by mass; and
particularly preferably, 0 to 91% by mass/5 to 30% by mass/3 to 30% by mass/1 to 30% by mass/0.01 to 3% by mass/0% by mass.

The content ratio of each component is selected from each range so that the summation thereof is 100% by mass. Through the above content ratio, there is a tendency that performance such as sensitivity, resolution and developability is excellent. A "solid component" refers to a component except for the solvent. The "total mass of the solid component" means that the total of components excluding the solvent from the components constituting the composition is 100% by mass.

The composition of the present embodiment is normally prepared by dissolving each component in a solvent upon use into a homogeneous solution, and then if required, filtering through a filter or the like with a pore diameter of about 0.2 µm, for example.

### [Physical properties and the like of composition]

The composition of the present embodiment can form an amorphous film by spin coating. Also, the composition of the present embodiment can be applied to a general semiconductor production process. In addition, any of positive type and negative type resist patterns can be individually prepared from the composition of the present embodiment, depending on the kind of a developing solution to be used.

In the case of a positive type resist pattern, the dissolution rate of an amorphous film formed by spin coating with the composition of the present embodiment in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, there is a tendency that the amorphous film can be made into a resist that is insoluble in the developing solution. In addition, when the amorphous film has a dissolution rate of 0.0005 angstrom/sec or more, the resolution may be improved. It is presumed that this is because due to the change in the solubility before and after exposure of the base material (A), contrast at the interface between the exposed portion being dissolved in the developing solution and the unexposed portion not being dissolved in the developing solution is increased. Also, when the amorphous film has a dissolution rate of 0.0005 angstrom/sec or more, the effect of reducing LER and the effect of reducing defects tend to be obtained.

In the case of a negative type resist pattern, the dissolution rate of an amorphous film formed by spin coating with the composition of the present embodiment in a developing solution at 23°C is preferably 10 angstrom/sec or more. When the dissolution rate is 10 angstrom/sec or more, the amorphous film is easily dissolved in a developing solution, and is more suitable for a resist. In addition, when the amorphous film has a dissolution rate of 10 angstrom/sec or more, the resolution may be improved. It is presumed that this is because the micro surface portion of the base material (A) is dissolved, and LER is reduced. Also, when the amorphous film has a dissolution rate of 10 angstrom/sec or more, the effect of reducing defects tends to be obtained.

The dissolution rate can be determined by immersing the amorphous film in a developing solution for a predetermined period of time at 23°C and then measuring the film thickness before and after immersion by a publicly known method such as visual, ellipsometric, or QCM method.

In the case of a positive type resist pattern, the dissolution rate of a portion, exposed by radiation such as KrF excimer laser, extreme ultraviolet, electron beam or X-ray, of the amorphous film formed by spin coating with the composition of the present embodiment in a developing solution at 23°C is preferably 10 angstrom/sec or more. When the dissolution rate is 10 angstrom/sec or more, the amorphous film is easily dissolved in a developing solution, and is more suitable for a resist. In addition, when the amorphous film has a dissolution rate of 10 angstrom/sec or more, the resolution may be improved. It is presumed that this is because the micro surface portion of the base material (A) is dissolved, and LER is reduced. Also, when the dissolution rate is 10 angstrom/sec or more, the effect of reducing defects tends to be obtained.

In the case of a negative type resist pattern, the dissolution rate of a portion, exposed by radiation such as KrF excimer laser, extreme ultraviolet, electron beam or X-ray, of the amorphous film formed by spin coating with the composition of the present embodiment, in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, the amorphous film can be made into a resist that is insoluble in the developing solution. In addition, when the amorphous film has a dissolution rate of 0.0005 angstrom/sec or more, the resolution may be improved. It is presumed that this is because due to the change in the solubility before and after exposure of the base material (A), contrast at the interface between the unexposed portion being dissolved in the developing solution and the exposed portion not being dissolved in the developing solution is increased. Also, when the amorphous film has a dissolution rate of 0.0005 angstrom/sec or more, the effect of reducing LER and the effect of reducing defects tend to be obtained.

### [Method for producing amorphous film]

The composition of the present embodiment can be used to form an amorphous film on a substrate.

### [Resist pattern formation method using composition]

A method for forming a resist pattern, using the composition of the present embodiment, comprises the steps of: forming a photoresist layer on a substrate, using the composition of the present embodiment mentioned above; irradiating a predetermined region of the photoresist layer formed on the substrate with radiation; and developing the photoresist layer after the radiation irradiation.

A method for forming a resist pattern, using the composition of the present embodiment, comprises the steps of: forming a resist film on a substrate, using the composition of the present embodiment mentioned above; exposing at least a portion of the formed resist film; and developing the exposed resist film, thereby forming a resist pattern. The resist pattern according to the present embodiment can also be formed as an upper layer resist in a multilayer process.

Examples of the resist pattern formation method include, but not particularly limited to, the following method. A resist film (photoresist layer) is formed by coating a conventionally publicly known substrate with the composition of the present embodiment using a coating means such as spin coating, flow casting coating and roll coating. Examples of the conventionally publicly known substrate can include, but not particularly limited to, a substrate for electronic components, and the one having a predetermined wiring pattern formed thereon, or the like. More specific examples thereof include a silicon wafer, a substrate made of a metal such as copper, chromium, iron and aluminum, and a glass substrate. Examples of the wiring pattern material include copper, aluminum, nickel and gold. Also, if required, the substrate mentioned above may have an inorganic and/or organic film provided thereon. Examples of the inorganic film include an inorganic antireflection film (inorganic BARC). Examples of the organic film include an organic antireflection film (organic BARC). Surface treatment with hexamethylene disilazane or the like may be conducted.

Next, the coated substrate is heated, if required. The heating conditions vary according to the compounding composition of the composition or the like, but are preferably 20 to 250°C, and more preferably 20 to 150°C. By heating, the adhesiveness of the composition to the substrate may be improved, which is preferable. Then, the resist film is exposed to a desired pattern by any radiation selected from the group consisting of visible light, ultraviolet, excimer laser, electron beam, extreme ultraviolet (EUV), X-ray and ion beam. The exposure conditions or the like are arbitrarily selected according to the compounding composition of the composition or the like. In the present embodiment, in order to stably form a fine pattern with a high degree of accuracy in exposure, the resist film is preferably heated after radiation irradiation.

Next, by developing the exposed resist film in a developing solution, a predetermined resist pattern is formed. As the developing solution, it is preferable to select a solvent having a solubility parameter (SP value) close to that of the base material (A) to be used. For example, a polar solvent such as a ketone-based solvent, an ester-based solvent, an alcohol-based solvent, an amide-based solvent, and an ether-based solvent, and a hydrocarbon-based solvent, or an alkaline aqueous solution, described in International Publication No. WO 2013/24778 can be used.

A plurality of above solvents may be mixed, or the solvent may be used by mixing the solvent with a solvent other than those described above or water within the range having performance. However, in order to sufficiently exhibit the effect of the present embodiment, the water content ratio as the whole developing solution is less than 70% by mass, and is preferably less than 50% by mass, more preferably less than 30% by mass, and still more preferably less than 10% by mass. Particularly preferably, the developing solution is substantially moisture free. That is, the content of the organic solvent in the developing solution is 30% by mass or more and 100% by mass or less based on the total amount of the developing solution, preferably 50% by mass or more and 100% by mass or less, more preferably 70% by mass or more and 100% by mass or less, still more preferably 90% by mass or more and 100% by mass or less, and particularly preferably 95% by mass or more and 100% by mass or less.

Particularly, as the developing solution, a developing solution containing at least one kind of solvent selected from a ketone-based solvent, an ester-based solvent, an alcohol-based solvent, an amide-based solvent, and an ether-based solvent is preferable because it improves resist performance such as resolution and roughness of the resist pattern.

The vapor pressure of the developing solution is preferably 5 kPa or less at 20°C, further preferably 3 kPa or less, and particularly preferably 2 kPa or less. There is a tendency that the evaporation of the developing solution on the substrate or in a developing cup is inhibited by setting the vapor pressure of the developing solution to 5 kPa or less, to improve temperature uniformity within a wafer surface, thereby resulting in improvement in size uniformity within the wafer surface.

Specific examples of the developing solution having a vapor pressure of 5 kPa or less include those described in International Publication No. WO 2013/24778.

Specific examples of the developing solution having a vapor pressure of 2 kPa or less, which is a particularly preferable range, include those described in International Publication No. WO 2013/24778.

To the developing solution, a surfactant can be added in an appropriate amount, if required. The surfactant is not particularly limited, but an ionic or nonionic, fluorine-based and/or silicon-based surfactant or the like can be used, for example. Examples of the fluorine-based and/or silicon-based surfactant can include the surfactants described in Japanese Patent Laid-Open Nos. 62-36663, 61-226746, 61-226745, 62-170950, 63-34540, 7-230165, 8-62834, 9-54432, and 9-5988, and U.S. Pat. Nos. 5,405,720, 5,360,692, 5,529,881, 5,296,330, 5,436,098, 5,576,143, 5,294,511, and 5,824,451. The surfactant is preferably a nonionic surfactant. The nonionic surfactant is not particularly limited, but it is further preferable to use a fluorine-based surfactant or a silicon-based surfactant.

The amount of the surfactant used is usually 0.001 to 5% by mass based on the total amount of the developing solution, preferably 0.005 to 2% by mass, and further preferably 0.01 to 0.5% by mass.

For the development method, for example, a method for dipping a substrate in a bath filled with a developing solution for a fixed time (dipping method), a method for raising a developing solution on a substrate surface by the effect of a surface tension and keeping it still for a fixed time, thereby conducting the development (puddle method), a method for spraying a developing solution on a substrate surface (spraying method), and a method for continuously ejecting a developing solution on a substrate rotating at a constant speed while scanning a developing solution ejecting nozzle at a constant rate (dynamic dispense method), or the like may be applied. The time for carrying out the pattern development is not particularly limited, but is preferably 10 seconds to 90 seconds.

In addition, after the step of conducting the development, a step of stopping the development by the replacement with another solvent may be practiced.

After the development, it is preferable that a step of rinsing the resist film with a rinsing solution containing an organic solvent be included.

The rinsing solution used in the rinsing step after the development is not particularly limited as long as the rinsing solution does not dissolve the resist pattern cured by crosslinking. A solution containing a general organic solvent or water may be used as the rinsing solution. As the foregoing rinsing solution, a rinsing solution containing at least one kind of organic solvent selected from a hydrocarbon-based solvent, a ketone-based solvent, an ester-based solvent, an alcohol-based solvent, an amide-based solvent, and an ether-based solvent is preferably used. More preferably, after development, a step of rinsing the film by using a rinsing solution containing at least one kind of organic solvent selected from the group consisting of a ketone-based solvent, an ester-based solvent, an alcohol-based solvent and an amide-based solvent is conducted. Still more preferably, after development, a step of rinsing the film by using a rinsing solution containing an alcohol-based solvent or an ester-based solvent is conducted. Still more preferably, after the development, a step of rinsing the film by using a rinsing solution containing a monohydric alcohol is conducted. Particularly preferably, after the development, a step of rinsing the film by using a rinsing solution containing a monohydric alcohol having 5 or more carbon atoms is carried out. The time for rinsing the pattern is not particularly limited, but is preferably 10 seconds to 90 seconds.

Here, examples of the monohydric alcohol used in the rinsing step after the development include a linear, branched or cyclic monohydric alcohol. For example, those described in International Publication No. WO 2013/24778 can be used. As the monohydric alcohol having 5 or more carbon atoms, 1-hexanol, 2-hexanol, 4-methyl-2-pentanol, 1-pentanol, or 3-methyl-1-butanol is particularly preferable.

A plurality of these components may be mixed, or the component may be used by mixing the component with an organic solvent other than those described above.

The water content ratio in the rinsing solution is preferably 10% by mass or less, more preferably 5% by mass or less, and still more preferably 3% by mass or less. When the water content ratio is 10% by mass or less, there is a tendency that a better development characteristic can be obtained.

The vapor pressure at 20°C of the rinsing solution used after the development is preferably 0.05 kPa or more and 5 kPa or less, more preferably 0.1 kPa or more and 5 kPa or less, and most preferably 0.12 kPa or more and 3 kPa or less. By setting the vapor pressure of the rinsing solution to 0.05 kPa or more and 5 kPa or less, the temperature uniformity in the wafer surface is enhanced more, and furthermore, swelling due to permeation of the rinsing solution is further inhibited. As a result, the dimensional uniformity in the wafer surface is further improved.

The rinsing solution may also be used after adding an appropriate amount of a surfactant to the rinsing solution.

In the rinsing step, the wafer after the development is rinsed using the above organic solvent containing rinsing solution. The method for the rinsing treatment is not particularly limited. However, for example, a method for continuously ejecting a rinsing solution on a substrate spinning at a constant speed (spin coating method), a method for dipping a substrate in a bath filled with a rinsing solution for a fixed time (dipping method), and a method for spraying a rinsing solution on a substrate surface (spraying method), or the like can be applied. Above all, it is preferable to carry out the rinsing treatment by the spin coating method and after the rinsing, spin the substrate at a rotational speed of 2,000 rpm to 4,000 rpm, to remove the rinsing solution from the substrate surface.

After forming the resist pattern, a pattern wiring substrate is obtained by etching. Etching can be conducted by a publicly known method such as dry etching using plasma gas, and wet etching with an alkaline solution, a cupric chloride solution, and a ferric chloride solution or the like.

After forming the resist pattern, plating can also be conducted. Examples of the plating method include copper plating, solder plating, nickel plating, and gold plating.

The remaining resist pattern after the etching can be stripped by a publicly known method such as dry stripping and wet stripping. As the dry stripping, the remaining resist pattern can be stripped through photo-excited ashing in which ozone or oxygen gas is irradiated with light such as ultraviolet and resist stripping is performed using a chemical reaction between the gas and the resist, or through plasma ashing in which a gas is made into plasma with high frequency and the resist is stripped by utilizing that plasma. As the wet stripping, the remaining resist pattern can be stripped with a resist stripper or an organic solvent. Examples of the organic solvent include PGMEA (propylene glycol monomethyl ether acetate), PGME (propylene glycol monomethyl ether), and EL (ethyl lactate). Examples of the peeling method include, for example, a dipping method and a spraying method. A wiring substrate having a resist pattern formed thereon may be a multilayer wiring substrate, and may have a small diameter through hole.

In the present embodiment, the wiring substrate can also be formed by a method for forming a resist pattern, then depositing a metal in vacuum, and subsequently dissolving the resist pattern in a solution, that is, a liftoff method.

### [Insulating film formation method]

Also, in the present embodiment, in the same way as the resist pattern formation method mentioned above, an insulating film can be formed through the steps of: forming a photoresist layer on a substrate, using the composition of the present embodiment; irradiating a predetermined region of the photoresist layer formed on the substrate with radiation; and developing the photoresist layer after the radiation irradiation.

### Examples

The present embodiment will be described in more detail with reference to synthesis examples and examples below. However, the present embodiment is not limited to these examples by any means.

### [Measurement method]

### Structure of compound

The structure of the compound was verified by carrying out ¹H-NMR measurement under the following conditions using a product from Bruker, Advance 600 II spectrometer.
Frequency: 400 MHz
Solvent: d6-DMSO
Internal standard: TMS
Measurement temperature: 23°C

### [Evaluation method]

### (1) Safe solvent solubility test of compound

The solubility of the compounds in PGME, PGMEA, and CHN was evaluated according to the following criteria utilizing the amount of dissolution in each solvent. The amount of dissolution was measured at 23°C by precisely weighing the compound into a test tube, adding the solvent of interest so as to attain a predetermined concentration, applying ultrasonic waves for 30 minutes in an ultrasonic cleaner, and then visually observing the subsequent state of the fluid.
A: 5.0% by mass ≤ Amount of dissolution
B: 2.0% by mass ≤ Amount of dissolution < 5.0% by mass
C: Amount of dissolution < 2.0% by mass

### (2) Storage stability and thin film formability of resist composition

The storage stability of the resist composition containing the compound was evaluated by leaving the resist composition after preparation to stand still at 23°C for 3 days, and visually observing the presence or absence of precipitates. A clean silicon wafer was spin coated with the resist composition, and then prebaked (PB) before exposure on a hot plate at 110°C to form a resist film with a thickness of 50 nm. The prepared resist composition was evaluated as ○ when it was a homogeneous solution and formed a good thin film, as Δ when it was a homogeneous solution and there were defects in the thin film, and as × when there were precipitates.

### (3) Pattern evaluation of resist pattern (pattern formation)

The resist film obtained in the above (2) was irradiated with electron beams of 1:1 line and space setting with a 50 nm interval using an electron beam lithography system (ELS-7500 manufactured by ELIONIX INC.).

After irradiation, the resist film was heated at 110°C for 90 seconds, and immersed in a 2.38 mass% TMAH alkaline developing solution for 60 seconds for development. Then, the resist film was washed with ultrapure water for 30 seconds, and dried to form a resist pattern.

The shape of the obtained resist patterns of 50 nmL/S (1:1) was observed using an electron microscope manufactured by Hitachi Ltd. (S-4800). With respect to the "resist pattern shape" after the development, those having no pattern collapse and better rectangularity than Comparative Example 1 were evaluated to be "A", and those having a rectangularity equivalent to or inferior to Comparative Example 1 were evaluated to be "C". Furthermore, the smallest electron beam energy quantity capable of lithographing a good pattern shape was set as "sensitivity", and those in which the sensitivity is superior to Comparative Example 1 by 10% or more were evaluated to be "S", those in which the sensitivity is superior by less than 10% were evaluated to be "A", and those in which the sensitivity is equivalent to or inferior to Comparative Example 1 were evaluated to be "C".

### (4) Etching resistance

Etching apparatus: RIE-10NR manufactured by Samco International, Inc.
Output: 50 W
Pressure: 20 Pa
Time: 2 min
Etching gas
Ar gas flow rate:CF₄ gas flow rate:O₂ gas flow rate = 50:5:5 (sccm)

For each of the films obtained in Examples and Comparative Example, the etching test was carried out under the conditions mentioned above, and the etching rate upon that time was measured. Then, the etching resistance was evaluated according to the following evaluation criteria on the basis of the etching rate of an underlayer film prepared by using a novolac ("PSM4357" manufactured by Gunei Chemical Industry Co., Ltd.).

### Evaluation criteria

A: The etching rate was smaller as compared with the underlayer film of novolac.
C: The etching rate was larger as compared with the underlayer film of novolac.

### [Base material (A)]

### Synthesis Comparative Example 1 (Synthesis of acrylic polymer 1)

4.15 g of 2-methyl-2-methacryloyloxyadamantane, 3.00 g of methacryloyloxy-γ-butyrolactone, 2.08 g of 3-hydroxy-1-adamantyl methacrylate, and 0.38 g of azobisisobutyronitrile were dissolved in 80 mL of tetrahydrofuran to prepare a reaction solution. This reaction solution was polymerized for 22 hours with the reaction temperature kept at 63°C in a nitrogen atmosphere. Then, the reaction solution was added dropwise into 400 mL of n-hexane. The obtained product resin was solidified and purified, and the resulting white powder was filtered and then dried overnight at 40°C under reduced pressure to obtain an acrylic polymer 1 represented by the following formula (11).

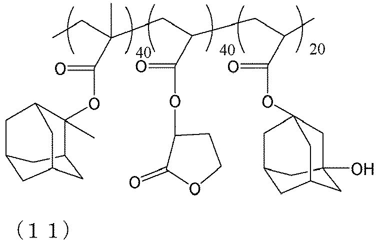

In the above formula (11), "40", "40", and "20" represent the ratio of each constituent unit and do not represent a block copolymer.

### (Production of polyphenols (B))

### Synthesis Example 1: Synthesis of MGR216

To a container (internal capacity: 500 mL) equipped with a stirrer, a condenser tube, and a burette, 42.8 g (230 mmol) of 4,4'-biphenol (a reagent manufactured by Tokyo Chemical Industry Co., Ltd.), 21.5 g (57.5 mmol) of 3,5-diiodosalicylaldehyde (a reagent manufactured by Tokyo Chemical Industry Co., Ltd.), and 428 mL of γ-butyrolactone were charged, and 5.8 g (58 mmol) of sulfuric acid was added thereto to prepare a reaction liquid. This reaction liquid was reacted with stirring at 90°C for 56 hours. After the reaction finished, 1L of pure water was added to the reaction liquid, which was then neutralized with sodium hydroxide. Extraction with ethyl acetate was performed, followed by concentration, to obtain a solution. The obtained solution was separated and purified by column chromatography to obtain 10 g of the objective compound (MGR216) represented by the following formula. The following peaks were found by NMR measurement performed on the obtained compound (MGR216) under the above measurement conditions, and the compound was confirmed to have a chemical structure of the following formula (MGR216).
δ (ppm) 9.4 (4H, -O-H), 8.9 (1H, -O-H), 6.2-7.8 (16H, Ph-H), 6.3 (1H, C-H)

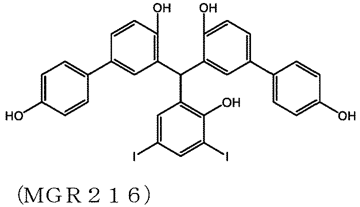

### Synthesis Example 2: Synthesis of MGR216-MeBOC

To a container (internal capacity: 500 mL) equipped with a stirrer, a condenser tube, and a burette, 9.0 g (12.4 mmol) of the compound (MGR216) obtained as described above, 13.5 g (68 mmol) of t-butyl bromoacetate (manufactured by Sigma-Aldrich), and 200 mL of acetone were charged, 9.5 g (68 mmol) of potassium carbonate (manufactured by Sigma-Aldrich) and 1.0 g of 18-crown-6 were added thereto, and the contents were reacted with stirring for 3 hours under reflux to obtain a reaction liquid. Next, the reaction liquid was concentrated, and the reaction product was precipitated by the addition of 200 g of pure water to the concentrate, cooled to room temperature, and then filtered to separate solid matter.

The obtained solid matter was dried, and then separated and purified by column chromatography to obtain 1.5 g of the following formula (MGR216-MeBOC).

The following peaks were found by NMR measurement performed on the obtained compound (MGR216-MeBOC) under the above measurement conditions, and the compound was confirmed to have a chemical structure of the following formula (MGR216-MeBOC).
δ (ppm) 6.2-7.8 (16H, Ph-H), 6.3 (1H, C-H), 5.0 (10H, O-CH₂-C) , 1.4 (45H, O-C-CH₃)

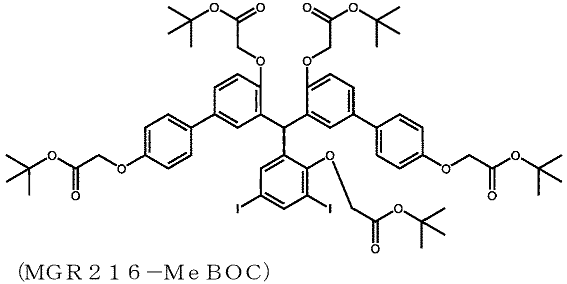

### Synthesis Example 3: Synthesis of X-27DHN4H35I

In a container (internal capacity: 300 mL) equipped with a stirrer, a condenser tube, and a burette, 7.0 g (40 mmol) of 2,7-naphthalenediol (a reagent manufactured by Sigma-Aldrich) and 7.5 g (20 mmol) of 4-hydroxy-3,5-diiodobenzaldehyde (a reagent manufactured by Tokyo Kasei Kogyo Co., Ltd.) were charged into 100 mL of γ-butyrolactone, 0.5 g of p-toluenesulfonic acid was added thereto, and the contents were reacted with stirring at 90°C for 23 hours to obtain a reaction liquid. Next, the reaction liquid was added to 1000 g of pure water, and then extracted with ethyl acetate and concentrated to obtain a solution.

The obtained solution was separated by column chromatography and washed with chloroform to obtain 0.5 g of the objective compound represented by the following formula (X-27DHN4H35I).

The following peaks were found by NMR measurement performed on the obtained compound (X-27DHN4H35I) under the above measurement conditions, and the compound was confirmed to have a chemical structure of the following formula (X-27DHN4H35I).
δ (ppm) 9.6 (1H, O-H), 9.2 (2H, O-H), 6.8-7.8 (12H, Ph-H), 5.3 (1H, C-H)

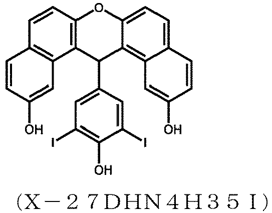

### Synthesis Example 4: Synthesis of MGR216-BOC

In a container (internal capacity: 200 mL) equipped with a stirrer, a condenser tube, and a burette, 9.1 g (12.5 mmol) of the compound (MGR216) obtained in Synthesis Example 1 and 13.7 g (62.8 mmol) of di-t-butyl dicarbonate (manufactured by Sigma-Aldrich) were charged into 100 mL of acetone, 8.64 g (62.5 mmol) of potassium carbonate (manufactured by Sigma-Aldrich) was added, and the contents were reacted with stirring at 20°C for 6 hours to obtain a reaction liquid. Next, the reaction liquid was concentrated, and the reaction product was precipitated by the addition of 100 g of pure water to the concentrate, cooled to room temperature, and then filtered to separate solid matter.

The obtained solid matter was filtered, dried, and then separated and purified by column chromatography, thereby obtaining 1.2 g of the objective compound (MGR216-BOC) represented by the following formula (MGR216-BOC).

As a result of measuring the molecular weight of the obtained compound (MGR216-BOC) by the above method, it was 716.

The following peaks were found by NMR measurement performed on the obtained compound (MGR216-BOC) under the above measurement conditions, and the compound was confirmed to have a chemical structure of the following formula (MGR216-BOC).
δ (ppm) 6.2-7.8 (16H, Ph-H), 6.3 (1H, C-H), 1.4 (45H, O-C-CH₃)

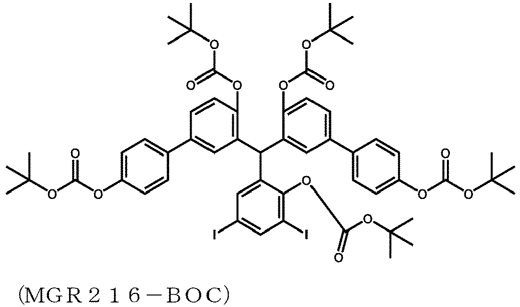

### Synthesis Example 5: Synthesis of MGR216-AL

To a container (internal capacity: 1000 mL) equipped with a stirrer, a condenser tube, and a burette, 9.1 g (12.5 mmol) of the compound (MGR216) obtained in Synthesis Example 1, 108 g (810 mmol) of potassium carbonate, and 200 mL of dimethylformamide were charged, 200 g (1.65 mol) of allyl bromide was added thereto, and the reaction liquid was reacted with stirring at 110°C for 24 hours. Next, the reaction liquid was concentrated. The reaction product was precipitated by the addition of 500 g of pure water. After cooling to room temperature, the precipitates were separated by filtration. The obtained solid matter was filtered and dried and then separated and purified by column chromatography to obtain 5.5 g of the objective compound (MGR216-AL) represented by the following formula.

The following peaks were found by NMR measurement performed on the obtained compound under the above measurement conditions, and the compound was confirmed to have a chemical structure of the following formula (MGR216-AL).
1H-NMR: (d6-DMSO, internal standard TMS):δ (ppm) 6.9-7.7 (16H, Ph-H), 6.3 (1H, C-H), 6.1 (5H, -CH=CH2), 5.3-5.4 (10H, -CH=CH2), 4.7 (10H, -CH2-)

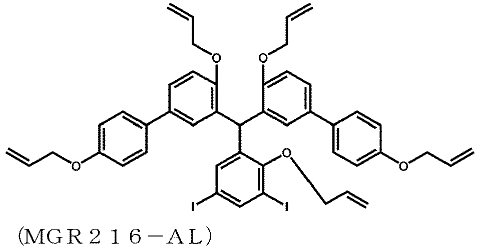

### Synthesis Example 6: Synthesis of MGR216-Ac

In the same manner as in Synthesis Example 5 except that 119 g (1.65 mol) of acrylic acid was used instead of 200 g (1.65 mol) of allyl bromide mentioned above, 5.3 g of MGR216-Ac represented by the following formula was obtained.

The following peaks were found by NMR measurement performed on the obtained compound under the above measurement conditions, and the compound was confirmed to have a chemical structure of the following formula (MGR216-Ac).
1H-NMR: (d6-DMSO, internal standard TMS):δ (ppm) 7.1-7.9 (16H, Ph-H), 6.2 (5H, =C-H), 6.1 (5H, -CH=C), 5.7 (5H, =C-H), 5.3 (1H, C-H)

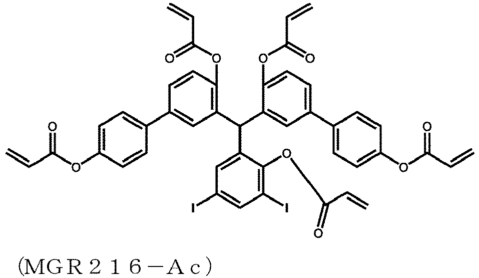

### Synthesis Example 7: Synthesis of MGR216-Ea

To a container (internal capacity: 100 mL) equipped with a stirrer, a condenser tube, and a burette, 7.3 g (10 mmol) of the compound (MGR216) obtained in Synthesis Example 1, 9.2 g of glycidyl methacrylate, 0.75 g of triethylamine, and 0.08 g of p-methoxyphenol were charged into 70 mL of methyl isobutyl ketone, and the contents were reacted with stirring for 24 hours while being warmed to 80°C and stirred.

The resultant was cooled to 50°C, and the reaction liquid was added dropwise into pure water. The precipitated solid matter was filtered, dried, and then separated and purified by column chromatography to obtain 2.0 g of the objective compound represented by the following formula (MGR216-Ea).

The obtained compound was confirmed to have a chemical structure of the following formula (MGR216-Ea) by 400 MHz-¹H-NMR.
1H-NMR: (d-DMSO, internal standard TMS)
δ (ppm) 7.0-7.9 (16H, Ph-H), 6.4-6.5 (10H, C=CH2), 5.5 (1H, C-H), 5.8 (5H, -OH), 4.7 (5H, C-H), 4.0-4.4 (20H, -CH2-), 2.0 (15H, -CH3)

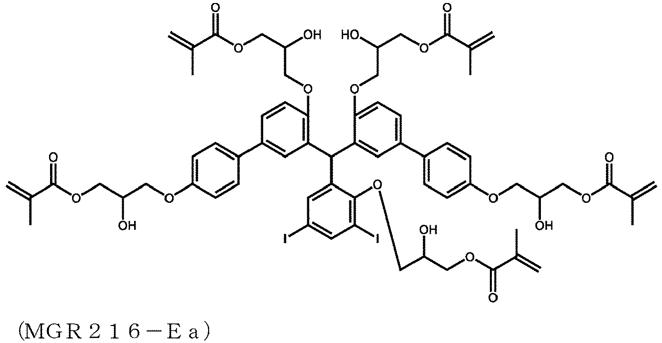

### Synthesis Example 8: Synthesis of MGR216-Ua

To a container (internal capacity: 100 mL) equipped with a stirrer, a condenser tube, and a burette, 7.3 g (10 mmol) of the compound (MGR216) obtained in Synthesis Example 1, 9.2 g of 2-isocyanatoethyl methacrylate, 0.75 g of triethylamine, and 0.08 g of p-methoxyphenol were charged into 70 mL of methyl isobutyl ketone, and the contents were reacted with stirring for 24 hours while being warmed to 80°C and stirred. The resultant was cooled to 50°C, and the reaction liquid was added dropwise into pure water. The precipitated solid matter was filtered, dried, and then separated and purified by column chromatography to obtain 1.9 g of the objective compound represented by the following formula (MGR216-Ua). The obtained compound was confirmed to have a chemical structure of the following formula (MGR216-Ua) by 400 MHz-¹H-NMR.
1H-NMR: (d-DMSO, internal standard TMS)
δ (ppm) 7.1-7.9 (16H, Ph-H), 6.8 (5H, -NH-), 6.4-6.5 (10H, =CH2), 5.5 (1H, C-H), 4.6 (10H, -CH2-), 3.2 (10H, - CH2-), 2.0 (15H, -CH3)

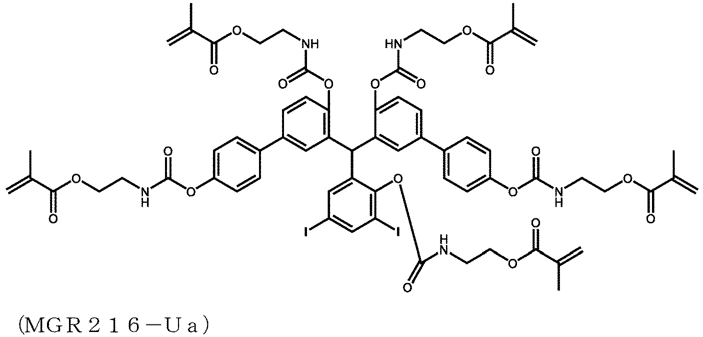

### Synthesis Example 9: Synthesis of MGR216-E

To a container (internal capacity: 100 mL) equipped with a stirrer, a condenser tube, and a burette, 7.3 g (10 mmol) of the compound (MGR216) obtained in Synthesis Example 1 and 18.0 g (130 mmol) of potassium carbonate were charged into 60 mL of dimethylformamide, 8.0 g (65 mmol) of acetic acid-2-chloroethyl was added, and the reaction liquid was reacted with stirring at 90°C for 12 hours. Next, the reaction liquid was cooled in an ice bath to precipitate crystals, which were then separated by filtration. Subsequently, to a container (internal capacity: 100 mL) equipped with a stirrer, a condenser tube, and a burette, 40 g of the crystals mentioned above, 40 g of methanol, and 100 g of THF and a 24% aqueous sodium hydroxide solution were charged, and the reaction liquid was reacted with stirring for 4 hours under reflux. Then, the reaction liquid was cooled in an ice bath and concentrated. The precipitated solid matter was filtered, dried, and then separated and purified by column chromatography to obtain 3.8 g of the objective compound represented by the following formula (MGR216-E). The obtained compound was confirmed to have a chemical structure of the following formula (MGR216-E) by 400 MHz-¹H-NMR.
1H-NMR: (d-DMSO, internal standard TMS)
δ (ppm) 7.0-8.0 (16H, Ph-H), 5.5 (1H, C-H), 4.9 (5H, -OH), 4.3 (10H, -CH2-), 3.7 (10H, -CH2-)

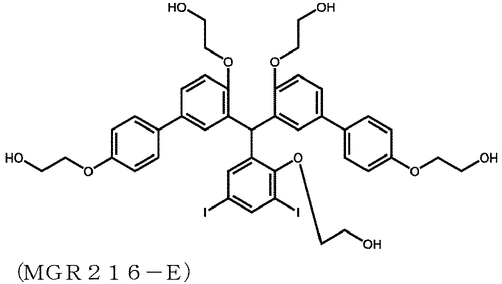

### Synthesis Example 10: Synthesis of MGR216-PX

To a container (internal capacity: 1000 mL) equipped with a stirrer, a condenser tube, and a burette, 72.5 g (42 mmol) of the compound (MGR216) obtained in Synthesis Example 1, 78.6 g of iodoanisole, 145.9 g of cesium carbonate, 2.35 g of dimethylglycine hydrochloride, and 0.85 g of copper iodide were charged into 400 mL of 1,4-dioxane, and the contents were warmed to 95°C and reacted with stirring for 22 hours. Next, insoluble matter was filtered off, and the filtrate was concentrated and added dropwise into pure water. The precipitated solid matter was filtered, dried, and then separated and purified by column chromatography to obtain 25 g of an intermediate compound represented by the following formula (MGR216-M).

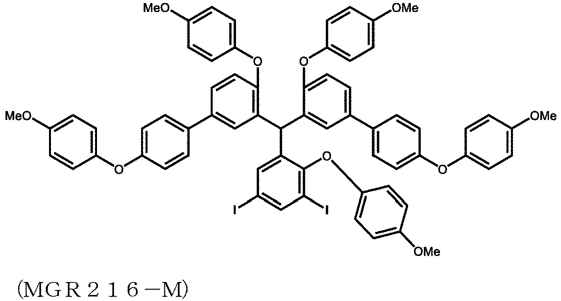

Next, to a container (internal capacity: 1000 mL) equipped with a stirrer, a condenser tube, and a burette, 10.6 g of the compound represented by the formula (MGR216-M) mentioned above and 80 g of pyridine hydrochloride were charged, and the contents were reacted with stirring at 190°C for 2 hours. Next, 160 mL of hot water was further added thereto, and the mixture was stirred to precipitate solid matter. Then, 250 mL of ethyl acetate and 100 mL of water were added thereto, and the mixture was stirred, left to stand still, and separated. The organic layer was concentrated, dried, and then separated and purified by column chromatography to obtain 6 g of the objective compound represented by the following formula (MGR216-PX).

The obtained compound was confirmed to have a chemical structure of the following formula (MGR216-PX) by 400 MHz-¹H-NMR.
1H-NMR: (d-DMSO, internal standard TMS)
δ (ppm) 9.5 (4H, O-H), 7.0-7.9 (36H, Ph-H), 5.5 (1H, C-H)

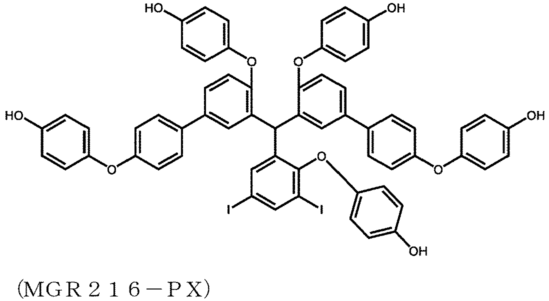

### Synthesis Example 11: Synthesis of MGR216-PE

The same reaction as in Synthesis Example 10 was performed except that the compound represented by the formula (MGR216-E) mentioned above was used instead of the compound represented by the formula (MGR216) mentioned above to obtain 4 g of the objective compound represented by the following formula (MGR216-PE).

The obtained compound was confirmed to have a chemical structure of the following formula (MGR216-PE) by 400 MHz-¹H-NMR.
1H-NMR: (d-DMSO, internal standard TMS)
δ (ppm) 9.1 (5H, O-H), 6.6-8.0 (36H, Ph-H), 5.5 (1H, C-H), 4.3 (10H, -CH2-), 3.1 (10H, -CH2-)

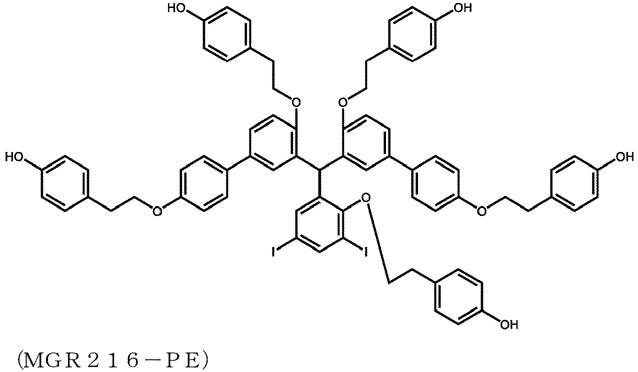

### Synthesis Example 12: Synthesis of MGR216-G

To a container (internal capacity: 100 mL) equipped with a stirrer, a condenser tube, and a burette, a solution containing 6.1 g (8.4 mmol) of the compound (MGR216) obtained in Synthesis Example 1 and 6.2 g (45 mmol) of potassium carbonate added to 100 mL of dimethylformamide was charged, 4.1 g (45 mmol) of epichlorohydrin was further added, and the reaction liquid was reacted with stirring at 90°C for 6.5 hours. Next, the solid component was then removed from the reaction liquid by filtration, and cooled in an ice bath to precipitate crystals, and the precipitated solid component was filtered, dried, and then separated and purified by column chromatography to obtain 2.2 g of the objective compound represented by the following formula (MGR216-G).

The following peaks were found by NMR measurement performed on the obtained compound (MGR216-G) under the measurement conditions mentioned above, and the compound was confirmed to have a chemical structure of the following formula (MGR216-G).
1H-NMR: (d-DMSO, internal standard TMS)
δ (ppm) 6.9-8.0 (16H, Ph-H), 5.5 (C-H), 3.9-4.2 (10H, - CH2-), 2.3-3.1 (15H, -CH (CH2) O)

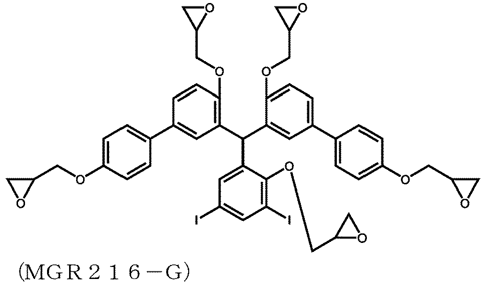

### Synthesis Example 13: Synthesis of MGR216-GE

The same reaction as in Synthesis Example 12 was performed except that the compound represented by the above formula (MGR216-E) was used instead of the compound represented by the above formula (MGR216) to obtain 1.5 g of the objective compound represented by the following formula (MGR216-GE).

The compound was confirmed to have a chemical structure of the following formula (MGR216-GE) by 400 MHz-¹H-NMR.
1H-NMR: (d-DMSO, internal standard TMS)
δ (ppm) 6.9-8.0 (16H, Ph-H), 5.5 (C-H), 3.3-4.3 (30H, - CH2-), 2.3-2.8 (15H, -CH (CH2) O)

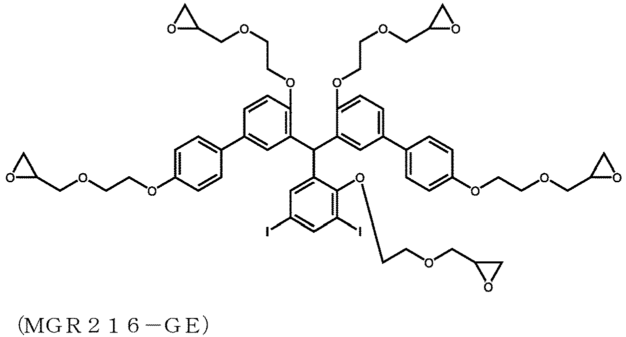

### Synthesis Example 14: Synthesis of MGR216-SX

To a container (internal capacity: 100 mL) equipped with a stirrer, a condenser tube, and a burette, 6.1 g (8.4 mmol) of the compound (MGR216) obtained in Synthesis Example 1 and 6.4 g of vinyl benzyl chloride (trade name CMS-P; manufactured by Seimi Chemical Co., Ltd.) were charged into 50 mL of dimethylformamide, and 8.0 g of 28 mass% sodium methoxide (methanol solution) was added from a dropwise funnel over 20 minutes while being warmed to 50°C and stirred, and the reaction liquid was reacted with stirring at 50°C for 1 hour. Next, 1.6 g of 28 mass% sodium methoxide (methanol solution) was added, and the reaction liquid was warmed to 60°C and stirred for 3 hours, 1.2 g of 85 mass% phosphoric acid was further added, and the mixture was stirred for 10 minutes, and then cooled to 40°C, and the reaction liquid was added dropwise into pure water. The precipitated solid matter was filtered, dried, and then separated and purified by column chromatography to obtain 2.1 g of the objective compound represented by the following formula (MGR216-SX).

The obtained compound was confirmed to have a chemical structure of the following formula (MGR216-SX) by 400 mhz-¹H-NMR.
1H-NMR: (d-DMSO, internal standard TMS)
δ (ppm) 7.0-7.9 (36H, Ph-H), 5.1-5.8 (21H, -CH2-, - C=CH, C-H), 6.7 (5H, -C=CH)

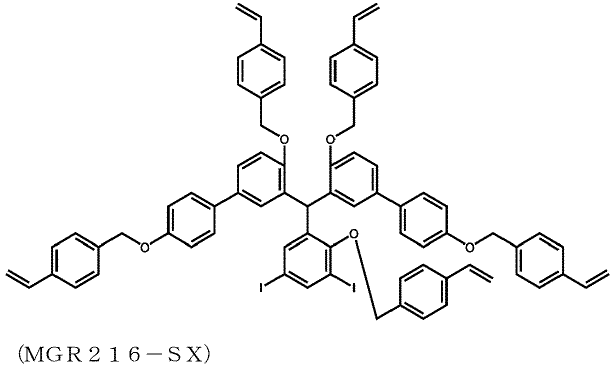

### Synthesis Example 15: Synthesis of MGR216-SE

The same reaction as in Synthesis Example 16 was performed except that the compound represented by the above formula (MGR216-E) was used instead of the compound represented by the above formula (MGR216) to obtain 2.0 g of the objective compound represented by the following formula (MGR216-SE).

The obtained compound was confirmed to have a chemical structure of the following formula (MGR216-SE) by 400 mhz-¹H-NMR.
1H-NMR: (d-DMSO, internal standard TMS)
δ (ppm) 7.0-8.0 (36H, Ph-H), 6.7 (5H, -CH=C), 5.8 (5H, -C=CH), 5.5 (1H, C-H), 5.3 (5H, -C=CH), 4.8 (10H, - CH2-), 4.3 (10H, -CH2-), 3.8 (10H, -CH2-)

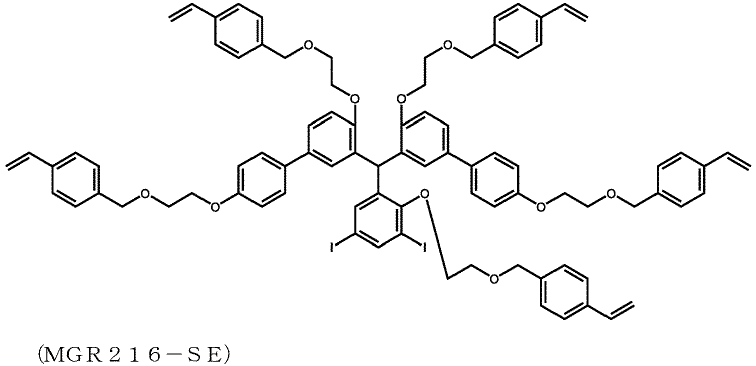

### Synthesis Example 16: Synthesis of MGR216-Pr

In a container (internal capacity: 300 mL) equipped with a stirrer, a condenser tube, and a burette, 6.1 g (8.4 mmol) of the compound (MGR216) obtained in Synthesis Example 1 and 7.9 g (66 mmol) of propargyl bromide were charged into 100 mL of dimethylformamide, and the contents were reacted with stirring at room temperature for 3 hours to obtain a reaction liquid. Next, the reaction liquid was concentrated, and the reaction product was precipitated by the addition of 300 g of pure water to the concentrate, cooled to room temperature, and then filtered to separate solid matter.

The obtained solid matter was filtered, dried, and then separated and purified by column chromatography, thereby obtaining 6.0 g of the objective compound (MGR216-Pr) represented by the following formula (MGR216-Pr) .

The following peaks were found by NMR measurement performed on the obtained compound (MGR216-Pr) under the measurement conditions mentioned above, and the compound was confirmed to have a chemical structure of the following formula (MGR216-Pr).
δ (ppm):7.0-8.0 (16H, Ph-H), 5.5 (1H, C-H), 4.7 (10H, -CH2-), 3.4 (5H, ≡CH)

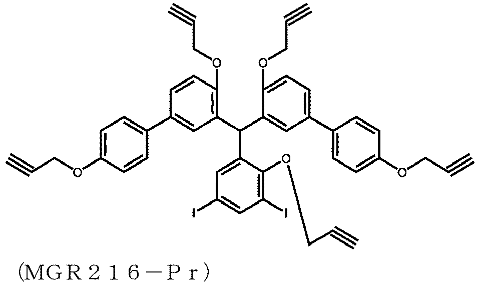

The results of evaluating the solubility of the compounds obtained in Synthesis Example 1 to 16 and Synthesis Comparative Example 1 in a safe solvent by the method described above are shown in Table 1.

**[Table 1]**

| | PGME | PGMEA | CHN |
|---|---|---|---|
| Synthesis Example 1 | A | A | A |
| Synthesis Example 2 | A | A | A |
| Synthesis Example 3 | A | A | A |
| Synthesis Example 4 | A | A | A |
| Synthesis Example 5 | A | A | A |
| Synthesis Example 6 | A | A | A |
| Synthesis Example 7 | A | A | A |
| Synthesis Example 8 | A | A | A |
| Synthesis Example 9 | A | A | A |
| Synthesis Example 10 | A | A | A |
| Synthesis Example 11 | A | A | A |
| Synthesis Example 12 | A | A | A |
| Synthesis Example 13 | A | A | A |
| Synthesis Example 14 | A | A | A |
| Synthesis Example 15 | A | A | A |
| Synthesis Example 16 | A | A | A |
| Comparative Production Example 1 | A | A | A |

### [Examples 1 to 20 and Comparative Example 1]

Compositions for lithography were each prepared according to the composition shown in Table 2 below. Next, a silicon substrate was spin coated with each of these compositions for lithography, and then baked at 110°C for 90 seconds to prepare each resist film with a film thickness of 50 nm. The following acid generating agent, acid diffusion controlling agent and organic solvent were used.

Acid generating agent: triphenylsulfonium nonafluoromethanesulfonate (TPS-109) manufactured by Midori Kagaku Co., Ltd.

Acid diffusion controlling agent: tri-n-octylamine (TOA) manufactured by Kanto Chemical Co., Inc.

Crosslinking agent: NIKALAC MW-100LM manufactured by Sanwa Chemical Co., Ltd.

Organic solvent: propylene glycol monomethyl ether (PGME) manufactured by Kanto Chemical Co., Inc.

**[Table 2]**

| | Resist base material (A) (parts by mass) | Polyphenol compound (B) (parts by mass) | Acid generating agent (parts by mass) | Acid diffusion controlling agent (parts by mass) | Crosslinking agent (parts by mass) | Organic solvent (parts by mass) |
|---|---|---|---|---|---|---|
| Example 1 | None | MGR216 | TPS-109 | TOA | MW-100LM | PGME |
| | | 5 | 1 | 0.1 | 1 | 120 |
| Example 2 | None | MGR216-MeBOC | TPS-109 | TOA | None | PGME |
| | | 5 | 1 | 0.1 | | 100 |
| Example 3 | Acrylic polymer 1 | MGR216 | TPS-109 | TOA | None | PGME |
| | 4.5 | 0.5 | 1 | 0.1 | | 100 |
| Example 4 | Acrylic polymer 1 | MGR216-MeBOC | TPS-109 | TOA | None | PGME |
| | 4.5 | 0.5 | 1 | 0.1 | | 100 |
| Example 5 | None | X-27DHN4H35I | TPS-109 | TOA | MW-100LM | PGME |
| | | 5 | 1 | 0.1 | 1 | 120 |
| Example 6 | None | MGR216-BOC | TPS-109 | TOA | None | PGME |
| | | 5 | 1 | 0.1 | | 100 |
| Example 7 | Acrylic polymer 1 | X- | TPS-109 | TOA | None | PGME |
| | 4.5 | 27DHN4H35I 0.5 | 1 | 0.1 | | 100 |
| Example 8 | Acrylic polymer 1 | MGR216-BOC | TPS-109 | TOA | None | PGME |
| | 4.5 | 0.5 | 1 | 0.1 | | 100 |
| Example 9 | None | MGR216-AL | TPS-109 | TOA | MW-100LM | PGME |
| | | 5 | 1 | 0.1 | 1 | 120 |
| Example 10 | None | MGR216-Ac | TPS-109 | TOA | MW-100LM | PGME |
| | | 5 | 1 | 0.1 | 1 | 120 |
| Example 11 | None | MGR216-Ea | TPS-109 | TOA | MW-100LM | PGME |
| | | 5 | 1 | 0.1 | 1 | 120 |
| Example 12 | None | MGR216-Ua | TPS-109 | TOA | MW-100LM | PGME |
| | | 5 | 1 | 0.1 | 1 | 120 |
| Example 13 | None | MGR216-E | TPS-109 | TOA | MW-100LM | PGME |
| | | 5 | 1 | 0.1 | 1 | 120 |
| Example 14 | None | MGR216-PX | TPS-109 | TOA | MW-100LM | PGME |
| | | 5 | 1 | 0.1 | 1 | 120 |
| Example 15 | None | MGR216-PE | TPS-109 | TOA | MW-100LM | PGME |
| | | 5 | 1 | 0.1 | 1 | 120 |
| Example 16 | None | MGR216-G | TPS-109 | TOA | MW-100LM | PGME |
| | | 5 | 1 | 0.1 | 1 | 120 |
| Example 17 | None | MGR216-GE | TPS-109 | TOA | MW-100LM | PGME |
| | | 5 | 1 | 0.1 | 1 | 120 |
| Example 18 | None | MGR216-SX | TPS-109 | TOA | MW-100LM | PGME |
| | | 5 | 1 | 0.1 | 1 | 120 |
| Example 19 | None | MGR216-SE | TPS-109 | TOA | MW-100LM | PGME |
| | | 5 | 1 | 0.1 | 1 | 120 |
| Example 20 | None | MGR216-Pr | TPS-109 | TOA | MW-100LM | PGME |
| | | 5 | 1 | 0.1 | 1 | 120 |
| Comparative Example 1 | Acrylic polymer 1 | None | TPS-109 | TOA | None | PGME |
| | 5 | | 1 | 0.1 | | 100 |

First, a silicon substrate was spin coated with each of the compositions of Examples 1 to 20 and Comparative Example 1, and then baked at 110°C for 90 seconds to prepare each resist film with a film thickness of 50 nm.

Then, each was evaluated by the above-described methods. The evaluation results are shown in Table 3.

**[Table 3]**

| | Storage stability and thin film formability | Resist pattern | Sensitivity | Etching resistance |
|---|---|---|---|---|
| Example 1 | ○ | A | S | A |
| Example 2 | ○ | A | S | A |
| Example 3 | ○ | A | A | A |
| Example 4 | ○ | A | A | A |
| Example 5 | ○ | A | S | A |
| Example 6 | ○ | A | S | A |
| Example 7 | ○ | A | A | A |
| Example 8 | ○ | A | A | A |
| Example 9 | ○ | A | S | A |
| Example 10 | ○ | A | S | A |
| Example 11 | ○ | A | S | A |
| Example 12 | ○ | A | S | A |
| Example 13 | ○ | A | S | A |
| Example 14 | ○ | A | S | A |
| Example 15 | ○ | A | S | A |
| Example 16 | ○ | A | S | A |
| Example 17 | ○ | A | S | A |
| Example 18 | ○ | A | S | A |
| Example 19 | ○ | A | S | A |
| Example 20 | ○ | A | S | A |
| Comparative Example 1 | ○ | C | C | C |

As mentioned above, the composition of the present embodiment can be used for a resist composition or the like that maintains good storage stability and thin film formability, has high sensitivity and high etching resistance, and provides a good resist pattern shape.

Therefore, the lithography composition and the pattern formation method can be utilized widely and effectively in a variety of applications that require such performances.

The present application is based on Japanese Patent Application No. 2018-157622 filed in the Japan Patent Office on August 24, 2018, the contents of which are incorporated herein by reference.

### Industrial applicability

The composition of the present invention has industrial applicability as a composition used for film formation purposes for resist.

## Claims

1. A composition comprising a polyphenol compound (B),
wherein the polyphenol compound (B) is one or more selected from the group consisting of a compound represented by the following formula (1) and a resin having a structure represented by the following formula (2) : 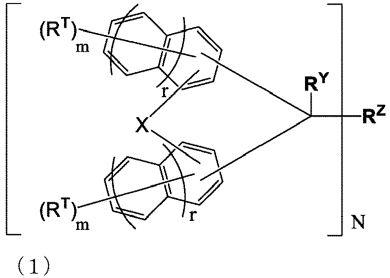
wherein R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms and optionally having a substituent, or a single bond;
each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
R^{z} contains at least two iodine atoms;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2); and
N is an integer of 1 to 4 (provided that when N is an integer of 2 or larger, N structural formulas within the parentheses [ ] are the same or different), and 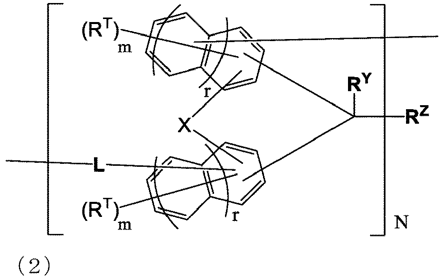 wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms and optionally having a substituent, or a single bond;
each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
R^{z} contains at least two iodine atoms;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2); and
N is an integer of 1 to 4 (provided that when N is an integer of 2 or larger, N structural formulas within the parentheses [ ] are the same or different).

2. The composition according to claim 1, further comprising a base material (A), wherein the base material (A) is one or more selected from the group consisting of a phenol novolac resin, a cresol novolac resin, a hydroxystyrene resin, a (meth)acrylic resin, a hydroxystyrene-(meth)acrylic copolymer, a cycloolefin-maleic anhydride copolymer, a cycloolefin, a vinyl ether-maleic anhydride copolymer and an inorganic resist material having a metallic element, and a derivative thereof.

3. The composition according to claim 1 or 2, wherein the polyphenol compound (B) is one or more selected from the group consisting of a compound represented by the following formula (1A) and a resin having a structure represented by the following formula (2A): 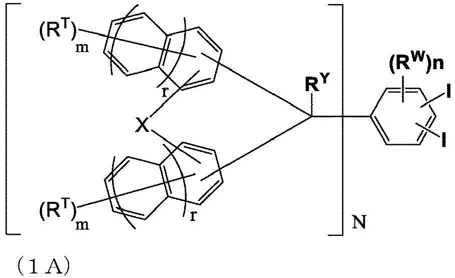
wherein R^{Y} is a hydrogen atom, an alkyl group having 1 to 30 carbon atoms and optionally having a substituent, or an aryl group having 6 to 30 carbon atoms and optionally having a substituent;
R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2);
each n is independently an integer of 0 to 3; and
N is an integer of 1 to 4 (provided that when N is an integer of 2 or larger, N structural formulas within the parentheses [ ] are the same or different), and 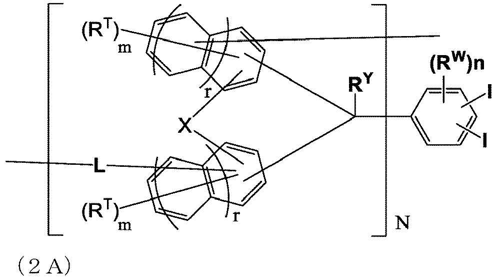
wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 30 carbon atoms and optionally having a substituent;
R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2);
each n is independently an integer of 0 to 3; and
N is an integer of 1 to 4 (provided that when N is an integer of 2 or larger, N structural formulas within the parentheses [ ] are the same or different).

4. The composition according to claim 2 or 3, wherein a mass ratio between the base material (A) and the polyphenol compound (B) is 5:95 to 95:5.

5. The composition according to any one of claims 1 to 4, further comprising a solvent.

6. The composition according to any one of claims 1 to 5, further comprising an acid generating agent.

7. The composition according to any one of claims 1 to 6, further comprising a crosslinking agent.

8. The composition according to any one of claims 1 to 7, wherein the composition is used in film formation for lithography.

9. The composition according to any one of claims 1 to 7, wherein the composition is used in film formation for resist.

10. A method for forming a resist pattern, comprising the steps of: forming a photoresist layer on a substrate using the composition according to any one of claims 1 to 7; irradiating a predetermined region of the photoresist layer formed on the substrate with radiation; and developing the photoresist layer after the radiation irradiation.

11. A method for forming an insulating film, comprising the steps of: forming a photoresist layer on a substrate using the composition according to any one of claims 1 to 7; irradiating a predetermined region of the photoresist layer formed on the substrate with radiation; and developing the photoresist layer after the radiation irradiation.

12. A compound represented by the following formula (1): 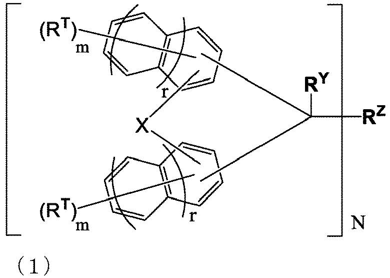
wherein R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms and optionally having a substituent, or a single bond;
each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
R^{z} contains at least two iodine atoms;
X is an oxygen atom, a sulfur atom or not a crosslink;
each r is independently an integer of 0 to 2 (provided that at least one r is an integer of 1 to 2);
each m is independently an integer of 0 to 9 (provided that at least one m is an integer of 1 to 9); and
N is an integer of 1 to 4 (provided that when N is an integer of 2 or larger, N structural formulas within the parentheses [ ] are the same or different).

13. A resin having a structure represented by the following formula (2): 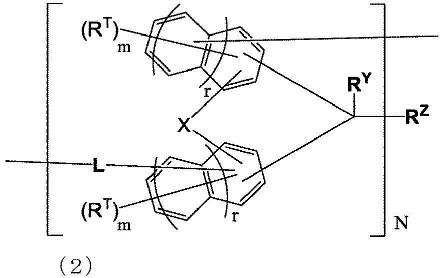
wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms and optionally having a substituent, or a single bond;
each R^{T} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
R^{z} contains at least two iodine atoms;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2); and
N is an integer of 1 to 4 (provided that when N is an integer of 2 or larger, N structural formulas within the parentheses [ ] are the same or different).

14. A compound represented by the following formula (1A) : 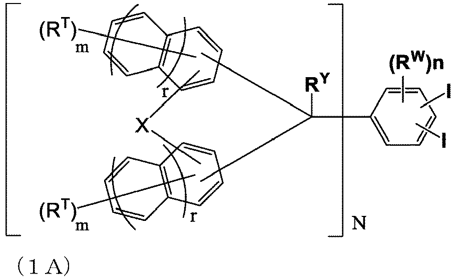
wherein R^{Y} is a hydrogen atom, an alkyl group having 1 to 30 carbon atoms and optionally having a substituent, or an aryl group having 6 to 30 carbon atoms and optionally having a substituent;
R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2);
each n is independently an integer of 0 to 3; and
N is an integer of 1 to 4 (provided that when N is an integer of 2 or larger, N structural formulas within the parentheses [ ] are the same or different).

15. A resin having a structure represented by the following formula (2A): 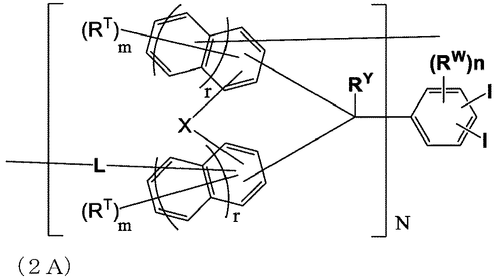
wherein L is an alkylene group having 1 to 30 carbon atoms and optionally having a substituent, an arylene group having 6 to 30 carbon atoms and optionally having a substituent, an alkoxylene group having 1 to 30 carbon atoms and optionally having a substituent, or a single bond, wherein the alkylene group, the arylene group and the alkoxylene group each optionally contain an ether bond, a ketone bond or an ester bond;
R^{Y} is a hydrogen atom, an alkyl group having 1 to 30 carbon atoms and optionally having a substituent, or an aryl group having 6 to 30 carbon atoms and optionally having a substituent;
R^{T} and R^{W} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a carboxylic acid group, a crosslinkable group, a dissociation group, a thiol group, or a hydroxy group (provided that at least one R^{T} contains a crosslinkable group, a dissociation group or a hydroxy group), wherein the alkyl group, the aryl group, the alkenyl group, the alkynyl group and the alkoxy group each optionally contain an ether bond, a ketone bond or an ester bond;
X is an oxygen atom, a sulfur atom or not a crosslink;
each m is independently an integer of 0 to 9 (provided that at least one m is an integer of 1 to 9);
each r is independently an integer of 0 to 2 (provided that at least one r is an integer of 1 to 2); each n is independently an integer of 0 to 3; and
N is an integer of 1 to 4 (provided that when N is an integer of 2 or larger, N structural formulas within the parentheses [ ] are the same or different).

16. A compound represented by the following formula (1B) :

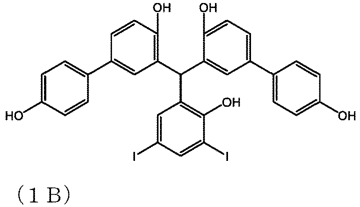

17. A compound represented by the following formula (2B) :

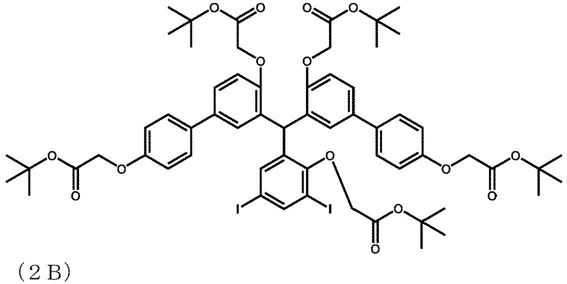

18. A compound represented by the following formula (2C) :
